# DEMANDE DE BREVET EUROPEEN

(11) **EP 1 757 660 A1**
(43) Date de publication de la demande: **28.02.2007**
(21) Numéro de dépôt: 06291333.0
(22) Date de dépôt: 22.08.2006
(51) Int. Cl.: C09B 56/12, C09B 43/36, A61K 8/49, A61Q 5/06, C09B 26/04, C09B 1/20

(54) **Colorants mixtes cationiques comprenant un chromophore anthraquinone et leur utilisation en colorant capillaire**

(30) Priorité: 26.08.2005 FR 0508793
(71) Demandeur: L'Oreal, 75008 Paris (FR)
(72) Inventeur: Daubresse, Nicolas, 78170 La Celle Saint Cloud (FR); Greaves, Andrew, 77144 Montevrain (FR); Berteuil, Nathalie, 91800 Brunoy (FR)
(74) Mandataire: Casalonga, Axel

(57) **Abrégé**

La présente invention a pour objet un colorant direct mixte cationique comprenant un chromophore anthraquinone et au moins un chromophore azoïque cationique et/ou au moins un chromophore hydrazone cationique, le chromophore anthraquinone étant lié à et/ou aux autres chromophores cationiques par l'intermédiaire d'au moins un bras de liaison.

L'invention porte également sur une composition tinctoriale comprenant ledit colorant direct et sur un procédé de coloration des fibres kératiniques, notamment humaines mettant en jeu ladite composition, ainsi qu'un dispositif approprié.

Elle concerne également l'utilisation desdits colorants et de ladite composition.

## Description

La présente invention est relative à de nouveaux colorants mixtes cationiques comprenant un chromophore anthraquinone et au moins un chromophore azoïque cationique et/ou au moins un chromophore hydrazone cationique, à une composition pour la coloration des fibres kératiniques, et en particulier des fibres kératiniques humaines telles que les cheveux, comprenant au moins un colorant mixte tel que défini ci-dessus, aux procédés de coloration mettant en oeuvre ladite composition, à des dispositifs à compartiments mettant en oeuvre ladite composition et à des utilisations dudit colorant et de ladite composition.

Il est connu de teindre les fibres kératiniques et en particulier les cheveux humains avec des compositions tinctoriales contenant des colorants directs. Ces composés sont des molécules colorées et colorantes ayant une affinité pour les fibres. Il est connu par exemple d'utiliser des colorants directs du type nitrés benzéniques, des colorants anthraquinoniques, des nitropyridines, des colorants du type azoïque, xanthénique, acridinique, azinique ou triarylméthane.

Habituellement, ces colorants sont appliqués sur les fibres, éventuellement en présence d'un agent oxydant, si l'on souhaite obtenir un effet simultané d'éclaircissement des fibres. Une fois le temps de pose écoulé, les fibres sont rincées, éventuellement lavées et séchées.

Les colorations qui résultent de l'utilisation de colorants directs sont des colorations temporaires ou semi-permanentes car la nature des interactions qui lient les colorants directs à la fibre kératinique, et leur désorption de la surface et/ou du coeur de la fibre sont responsables de leur faible puissance tinctoriale et de leur relative mauvaise tenue aux lavages ou à la transpiration.

La teinture de fibres kératiniques et notamment des cheveux peut être obtenue dans des teintes chromatiques, telles que le rouge ou l'orangé, concomitamment à un éclaircissement de la couleur naturelle du cheveu. En mélange, les colorants directs chromatiques permettent d'obtenir, également en conditions éclaircissantes, des nuances esthétiques naturelles. Cependant, l'évolution dans le temps des colorants, et tout particulièrement les shampooings successifs, altèrent la couleur, provoquant un virage indésirable de la couleur naturelle vers une couleur plus chromatique.

Le but de la présente invention est de fournir des colorants directs permettant l'obtention de nuances naturelles et ne présentant pas les inconvénients des colorants directs existants.

En particulier, l'un des buts de la présente invention est de fournir des colorants directs qui permettent d'obtenir des nuances variées sans problème de virage de la couleur dans le temps, tenaces notamment aux shampooings et non sélectifs, dans des formules éclaircissantes ou non.

Ces buts sont atteints par la présente invention qui a pour objet des nouveaux colorants directs mixtes cationiques comprenant un chromophore anthraquinone et au moins un chromophore azoïque cationique et/ou au moins un chromophore hydrazone cationique, le chromophore anthraquinone étant lié à et/ou aux autres chromophores cationiques par l'intermédiaire d'au moins un bras de liaison.

Ces colorants permettent de résoudre les difficultés rencontrées. Ils permettent d'obtenir des teintures de couleur naturelle, en conditions éclaircissantes, homogènes et tenaces, notamment aux shampooings.

L'invention a également pour objet une composition pour la teinture d'oxydation des fibres kératiniques, et en particulier des fibres kératiniques humaines telles que les cheveux, caractérisée par le fait qu'elle contient, dans un milieu approprié pour la teinture, au moins un colorant mixte tel que défini ci-dessus.

L'invention vise également un procédé de coloration des fibres kératiniques mettant en oeuvre une telle composition tinctoriale.

L'invention porte également sur un procédé de coloration éclaircissante des fibres kératiniques mettant en oeuvre une telle composition tinctoriale selon l'invention et une composition oxydante.

Un autre objet de l'invention consiste en l'utilisation des colorants mixtes définis ci-dessus pour la coloration capillaire.

L'utilisation des compositions contenant ces colorants mixtes est aussi envisagée.

L'invention porte enfin sur des dispositifs à compartiments ou « kits » comprenant ladite composition.

D'autres caractéristiques, aspects, objets et avantages de la présente invention apparaîtront encore plus clairement à la lecture de la description et des exemples qui suivent.

Dans ce qui va suivre et à moins qu'une indication plus précise ne soit donnée :

Un radical alkyle ou la partie alkyle d'un radical est dit(e) 'substitué(e)', lorsqu'il (elle) comprend au moins un substituant choisi parmi les groupements :
- hydroxyle,
- alcoxy en C₁-C₄, (poly)hydroxyalcoxy en C₂-C₄,
- alkylcarbonylamino en C₁-C₂,
- un groupement alcoxycarbonyle en C₁-C₄,
- un groupement alkylsulfinyle en C₁-C₄,
- un groupement alkylsulfonyle en C₁-C₄
- amino, amino substitué par un ou plusieurs groupements alkyle identiques ou différents en C₁-C₄, éventuellement porteur d'un ou plusieurs groupements identiques ou différents choisis parmi hydroxyle ou alcoxy en C₁-C₂, lesdits radicaux alkyle pouvant former avec l'atome d'azote auquel ils sont rattachés, un hétérocycle à 5 ou 6 chaînons, saturé ou non, éventuellement aromatique, choisi de préférence parmi la pyrrolidine, la pipérazine, l'homopipérazine, le pyrrole, l'imidazole, le pyrazole, ou comprenant éventuellement au moins un autre hétéroatome différent ou non de l'azote,

Un radical aryle ou hétéroaryle ou la partie aryle ou hétéroaryle d'un radical est dit(e) 'substitué(e)', lorsqu'il(elle) comprend au moins un substituant porté par un atome de carbone, choisi parmi :
- un radical alkyle en C₁-C₁₆, de préférence en C₁-C₈, éventuellement substitué par un ou plusieurs radicaux choisis parmi les radicaux hydroxy, alcoxy en C₁-C₂, (poly)-hydroxyalcoxy en C₂-C₄, acylamino, amino substitué par deux radicaux alkyles, identiques ou différents, en C₁-C₄, éventuellement porteurs d'au moins un groupement hydroxyle ou, les deux radicaux pouvant former avec l'atome d'azote auquel ils sont rattachés, un hétérocycle à 5 ou 7 chaînons, de préférence de 5 ou 6 chaînons, comprenant éventuellement un autre hétéroatome identique ou différent de l'azote ;
- un atome d'halogène tel que chlore, fluor ou brome ;
- un groupement hydroxyle ;
- un radical alcoxy en C₁-C₄ ; un radical (poly)-hydroxyalcoxy en C₂-C₄ ;
- un radical amino ; un radical amino substitué par un ou deux radicaux alkyles, identiques ou différents, en C₁-C₄ éventuellement porteurs d'au moins un groupement hydroxyle ou amino éventuellement substitué par deux radicaux alkyles en C₁-C₂ éventuellement substitués ou substitués par un groupement aryle éventuellement subtitué;
- un radical carbamoyle (R₅₃)₂N-CO-, dans lequel les radicaux R₅₃, identiques ou non, représentent un atome d'hydrogène, un radical alkyle en C₁-C₄ éventuellement porteur d'au moins un groupement hydroxyle ;
- un radical alkylsulfonylamino R₅₄SO₂-NR₅₅-, dans lequel le radical R₅₄ représente un radical alkyle en C₁-C₄ éventuellement porteur d'au moins un groupement hydroxyle, un radical phényle éventuellement porteur d'au moins un radical choisi parmi un radical alkyle en C₁-C₄, un radical hydroxyle, et le radical R₅₅ représente un radical alkyle en C₁-C₄, un radical phényle ;
- un radical aminosulfonyle (R₅₆)₂N-SO₂-, dans lequel les radicaux R₅₆, identiques ou non, représentent un atome d'hydrogène, un radical alkyle en C₁-C₄ éventuellement porteur d'au moins un groupement hydroxyle,
- un groupement alkylcarbonylamino R₅₇CO-NR₅₈-, dans lequel le radical R₅₇ représente un radical alkyle en C₁-C₄ et le radical R₅₈ représente un atome d'hydrogène ou un radical alkyle en C₁-C₄.

Cette définition s'applique notamment aux cycles aromatiques présents sur un motif anthraquinone.

La partie cyclique ou hétérocyclique d'un radical non aromatique est dit(e) substitué(e) lorsqu'il (elle) comprend au moins un substituant porté par un atome de carbone choisi parmi les groupements :
- hydroxyle,
- alcoxy en C₁-C₄, (poly)hydroxyalcoxy en C₂-C₄,
- alkylcarbonylamino ((R₅₇CO-NR₅₈-) dans lequel le radical R₅₇ est un radical alkyle en C₁-C₄ éventuellement porteur d'au moins un groupement hydroxyle et le radical R₅₈ est un atome d'hydrogène ou un radical alkyle en C₁-C₄,
- amino substitué par deux groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle à 5 ou 7 chaînons, comprenant éventuellement au moins un autre hétéroatome différent ou non de l'azote.

Lorsqu'un cycle ne porte pas le nombre maximum de substituants, alors la ou les positions non susbtituées portent un atome d'hydrogène.

En outre, les colorants mixtes selon l'invention étant cationiques, leur(s) contre-ion(s) sont choisis parmi les anions ou mélanges d'anions cosmétiquement acceptables, de nature minérale ou organique. A titre d'exemples d'anions, on peut citer notamment les halogénures, comme les chlorures, les bromures ; les hydroxydes ; les sulfates ; les hydrogénosulfates ; les carbonates, les perchlorates, les tétrafluoroborates, les hydrogénocarbonates, l'acétate ; le citrate ; le tartrate ; les alkylsulfates pour lesquels la partie alkyle, linéaire ou ramifiée, est en C₁-C₆, comme l'ion méthylsulfate, éthylsulfate ; les alkylsulfonates pour lesquels la partie alkyle, linéaire ou ramifiée, est en C₁-C₆; les arylsulfonates pour lesquels la partie aryle, de préférence phényle, est éventuellement substituée par un ou plusieurs radicaux alkyle en C₁-C₄, ou leurs mélanges.

Les colorants mixtes cationiques présents dans la composition selon l'invention vont tout d'abord être décrits.

Comme indiqué précédemment, les colorants mixtes cationiques comprennent un chromophore anthraquinone et au moins un chromophore azoïque cationique et/ou au moins un chromophore hydrazone cationique, le chromophore anthraquinone étant lié à et/ou à ou aux chromophores cationiques par l'intermédiaire d'au moins un bras de liaison.

Par chromophore, on entend un radical issu d'un colorant, c'est-à-dire un radical d'une molécule absorbant dans le domaine visible entre 400 et 800 nm. Il est à noter de plus que cette absorbance du colorant ne nécessite ni oxydation préalable de celui-ci, ni association avec d'autre(s) espèce(s) chimique(s).

Selon un mode de réalisation particulier de l'invention, le colorant mixte comprend deux ou trois chromophores.

Dans le cadre de l'invention, un chromophore est dit cationique lorsqu'il comprend au moins un atome d'azote quaternisé.

De plus, la ou les charges cationiques du ou des chromophores peuvent être engagées ou non dans un cycle.

Selon un mode de réalisation particulier de l'invention, le colorant mixte répond au composé de formule (Ia) ou (Ib): 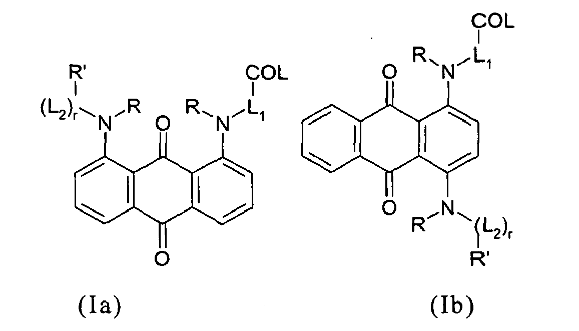 dans lesquelles :
- L₁ représente un bras de liaison, cationique ou non, reliant l'atome d'azote de l'anthraquinone au groupement COL par l'intermédiaire d'un atome de carbone, d'oxygène ou d'azote quaternisé ou non ;
- L₂ représente un bras de liaison, cationique ou non, reliant le deuxième azote de l'anthraquinone au groupement R' par l'intermédiaire d'un atome de carbone, d'oxygène ou d'azote quaternisé ou non ;
- r est égal à 0 ou 1 ;
- les groupements R représentent, indépendamment l'un de l'autre, un atome d'hydrogène ou un radical alkyle en C₁-C₄ éventuellement substitué ; le cas échéant, l'un au moins des groupements R peut être engagés dans un cycle saturé ou insaturé, éventuellement aromatique, comprenant de 5 à 7 chaînons, avec L₁ ou L₂ ;
- R' représente :
   - un atome d'hydrogène
   - une chaîne hydrocarbonée en C₁-C₁₂ linéaire ou ramifiée, éventuellement substituée, éventuellement interrompue ou terminée par un ou plusieurs groupements : amino, mono ou di-alkylamino éventuellement substitué, alkyl ou arylammonium, éventuellement substitué, hétérocycle à 5 ou 6 chaînons, saturé ou insaturé, comprenant au moins un atome d'azote quaternisé engagé dans ledit hétérocycle, ou
   - un groupement COL ;
- COL représente un radical colorant appartenant à la famille des azoïques cationiques ou des hydrazones cationiques,
   lorsque COL est un radical azoïque cationique, il répond à la formule générale suivante : 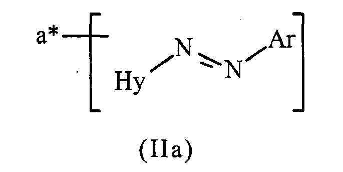
lorsque COL est un radical hydrazone cationique, il répond à la formule générale suivante : 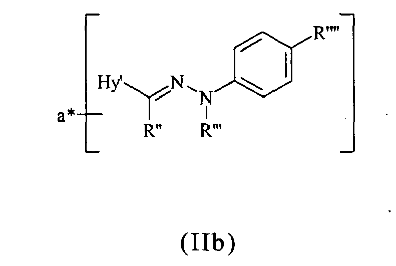
- a* représente la liaison reliant COL à L₁ ;
- Hy représente un hétérocycle cationique de formule générale : 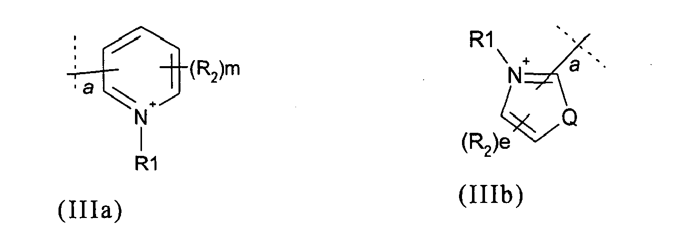
- Hy' représente un hétérocycle cationique de formule générale IIIa
- R₁ indépendamment les uns des autres représentent une chaîne hydrocarbonée en C₁-C₁₆, linéaire ou ramifiée, saturée ou insaturée, pouvant former un ou plusieurs cycles carbonés comportant de 5 à 7 chaînons, éventuellement condensés avec le cycle aromatique, éventuellement substituée, éventuellement interrompue par un ou plusieurs groupements choisis parmi les hétéroatomes tels que l'oxygène, l'azote ou le soufre, et le groupement carbonyle; R₁ ne comportant pas de fonction nitro, nitroso, peroxyde et diazo, R₁ étant directement rattaché à l'atome d'azote quaternisé ou non du cycle hétéroaromatique par l'intermédiaire d'un atome de carbone.
- R₂ indépendamment les uns des autres, représentent :

- une chaîne hydrocarbonée en C₁-C₁₆, linéaire ou ramifiée, saturée ou insaturée, aromatique ou non, pouvant former un ou plusieurs cycles carbonés comportant de 5 à 6 chaînons, éventuellement substituée, éventuellement interrompue par un ou plusieurs hétéroatomes ou par un ou plusieurs groupements portant au moins un hétéroatome, de préférence choisis parmi l'oxygène, l'azote, le soufre, tels que -CO-,
- SO₂- ou leurs combinaisons ;
- un groupement hydroxyle,
   - un groupement alcoxy en C₁-C₄ ; un groupement (poly)hydroxyalcoxy en C₂-C₄,
   - un groupement alcoxycarbonyle R₁₁O-CO-, dans lequel R₁₁ représente un radical alkyle en C₁-C₄ ; un radical alkylcarbonyloxy R₁₂CO-O-, dans lequel R₁₂ représente un radical alkyle en C₁-C₄ ;
   - un groupement amino ; un groupement amino substitué par un ou deux radicaux alkyles en C₁-C₄, identiques ou différents, éventuellement porteurs d'au moins un groupement hydroxyle, les deux radicaux alkyles formant éventuellement avec l'atome d'azote auquel ils sont rattachés, un hétérocycle à 5 ou 6 chaînons éventuellement porteur d'un autre hétéroatome identique ou différent de l'azote, par exemple l'oxygène, le soufre ;
   - un groupement alkylcarbonylamino R₁₃CO-NR₁₄-, dans lequel les radicaux R₁₃ et R₁₄, indépendamment l'un de l'autre, représentent un radical alkyle en C₁-C₄; un groupement carbamoyle (R₁₅)₂N-CO, dans lequel les radicaux R₁₅, indépendamment l'un de l'autre, identiques ou non, représentent un atome d'hydrogène, un radical alkyle en C₁-C₄; un groupement uréido (R₁₆)₂N-CO-NR₁₇-, dans lequel les radicaux R₁₆ et R₁₇, indépendamment les uns des autres, représentent un atome d'hydrogène, un radical alkyle en C₁-C₄ ;
   - un groupement sulfonamide (R₁₈)₂N-SO₂-, dans lequel les radicaux R₁₈, indépendamment l'un de l'autre, représentent un atome d'hydrogène, un radical alkyle en C₁-C₄; un groupement alkylsulfonylamino R₁₉SO₂-NR₂₀-, dans lequel les radicaux R₁₉ et R₂₀, indépendamment l'un de l'autre, représentent un atome d'hydrogène, un radical alkyle en C₁-C_{4;}
   - un groupement guanidinium (R₂₁)₂N-C(=NH₂⁺)-NR₂₂-, dans lequel les radicaux R₂₁ et R₂₂, indépendamment l'un de l'autre, représentent un atome d'hydrogène, un radical alkyle en C₁-C₄;
   - un groupement alkylsulfonyle R₂₃-SO₂-, dans lequel R₂₃ représente un radical alkyle en C₁-C₄ ;
   - un groupement nitro ; un groupement cyano ; un atome d'halogène, de préférence le chlore, le fluor ;
   - un groupement phényle éventuellement substitué ;
      deux radicaux R₂, portés par des atomes de carbone adjacents peuvent former ensemble avec l'atome de carbone auquel chacun est rattaché, un cycle condensé aromatique éventuellement substitué;
- m représente un entier compris entre 0 et 4 ; lorsque m est inférieur à 4, alors le ou les atomes de carbone de l'hétérocycle non substitués portent un atome d'hydrogène ;
- e est un entier compris entre 0 et 2 ; lorsque e est inférieur à 2, alors le ou les atomes de carbone de l'hétérocycle non substitués portent un atome d'hydrogène ;
- Q représente NR₁, O ou S ;
- la liaison α issue des formules (IIIa) ou (IIIb), relie le groupement Hy au groupement azoïque -N=N-Ar de la formule (IIa) ou le groupement Hy' au groupement hydrazone -CR"=N-NR"' de la formule (IIb);
   dans le cas des formules (IIIa) ou (IIIb), et lorsque deux radicaux R₂ portés par deux atomes de carbone adjacents forment un cycle aromatique, la liaison a peut relier le groupement Hy au groupement azoïque -N=N- de la formule (IIa) ou le groupement Hy' au groupement hydrazone -CR"=N-NR"'- de la formule (IIb) par l'intermédiaire dudit cycle aromatique ;
- Ar représente un cycle aromatique du type : 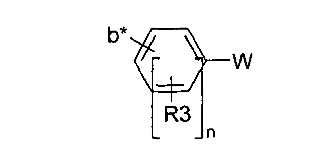
dans lequel:
- b* représente la liaison reliant Ar à NR"' de la formule (IIb) ou à la fonction azoïque de la formule (IIa) ;
- n est un nombre entier valant de 0 à 4 ; lorsque n est inférieur à 4, alors le ou les atomes de carbone du cycle aromatique non substitués portent un atome d'hydrogène ;

R₃ identiques ou différents, représentent :
- un radical alkyle en C₁-C₁₆, éventuellement substitué, éventuellement interrompu par un ou plusieurs hétéroatomes ou par un ou plusieurs groupements comprenant au moins un hétéroatome, de préférence choisi parmi l'oxygène, l'azote, le soufre, tels que -CO-,SO₂- ou leurs combinaisons ;
- un groupement hydroxyle,
- un groupement alcoxy en C₁-C₄ ; un groupement (poly)hydroxyalcoxy en C₂-C₄ ;
- un groupement alcoxycarbonyle R₃₁ O-CO-, dans lequel R₃₁ représente un radical alkyle en C₁-C₄ ; un radical alkylcarbonyloxy R₃₂CO-O-, dans lequel R₃₂ représente un radical alkyle en C₁-C₄ ;
- un radical alkylcarbonyle R₃₃-CO-, dans lequel R₃₃ représente un radical alkyle en C₁-C₄ ;
- un groupement amino ; un groupement amino substitué par un ou deux radicaux alkyle en C₁-C₄ , identiques ou différents, éventuellement porteurs d'au moins un groupement hydroxyle ; les deux radicaux alkyle formant éventuellement avec l'atome d'azote auquel ils sont rattachés, un hétérocycle renfermant 1 à 3 hétéroatomes, de préférence 1 à 2 hétéroatomes, choisis parmi N, O, S, de préférence N, comprenant de 5 à 7 chaînons, saturé ou insaturé, aromatique ou non, éventuellement substitué ;
- un groupement alkylcarbonylamino R₃₄CO-NR₃₅-, dans lequel le radical R₃₄ représente un radical alkyle en C₁-C₄ et le radical R₃₅ représente un atome d'hydrogène ou un radical alkyle en C₁-C₄ ;
- un groupement aminocarbonyle (R₃₆)₂N-CO-, dans lequel les radicaux R₃₆ indépendamment l'un de l'autre, représentent un atome d'hydrogène, un radical alkyle en C₁-C₄ ;
- un groupement uréido N(R₃₇)₂-CO-NR₃₈-, dans lequel les radicaux R₃₇ et R₃₈, indépendamment les uns des autres, représentent un atome d'hydrogène, un radical alkyle en C₁-C₄ ;
- un groupement aminosulfonyle (R₃₉)₂N-SO₂-, dans lequel les radicaux R₃₉ indépendamment l'un de l'autre, représentent un atome d'hydrogène, un radical alkyle en C₁-C₄ ;
- un groupement alkylsulfonylamino R₄₀SO₂-NR₄₁-, dans lequel les radicaux R₄₀ et R₄₁, indépendamment l'un de l'autre, représentent un atome d'hydrogène, un radical alkyle en C₁-C₄ ;
- un groupement thiol HS- ;
- un groupement alkylthio R₄₂S-, dans lequel le radical R₄₂ représente un radical alkyle en C₁-C₄ ;
- un groupement alkylsulfinyle R₄₃-SO-, dans lequel R₄₃ représente un radical alkyle en C₁-C₄ ;
- un groupement alkylsulfonyle R₄₄-SO₂-, dans lequel R₄₄ représente un radical alkyle en C₁-C₄ ;
- un groupement nitro ;
- un groupement cyano ;
- un atome d'halogène, de préférence le chlore, le fluor ;
lorsque n est supérieur ou égal à 2, deux radicaux R₃ adjacents peuvent former avec les atomes de carbone auxquels ils sont rattachés, un cycle secondaire, aromatique ou non à 6 chaînons, éventuellement substitué ;
W représente :
- un atome d'hydrogène,
- un atome d'halogène choisi parmi le brome, le chlore, le fluor de préférence le chlore et le fluor,
- un groupement -NR₅R₆, -OR₇, -NR₄-Ph-NR₅R₆, -NR₄-Ph-OR₇, -O-Ph-OR₇, -O-Ph-NR₅R₆, -SO₂-NR₅R₆, -SO₂-R₅ ; avec :

■ R₄, R₇, identiques ou non, représentent un atome d'hydrogène, un radical alkyle en C₁-C₂₀, de préférence en C₁-C₁₆ éventuellement substitué, un radical aryle ou aralkyle en C₆-C₃₀ éventuellement substitué;
■ R₅ et R₆ identiques ou non, représentent un atome d'hydrogène, un radical alkyle en C₁-C₂₀, de préférence en C₁-C₁₆ éventuellement substitué, un radical phényle éventuellement substitué, un radical aryle ou aralkyle en C₆-C₃₀ éventuellement substitué, un radical alkylcarbonyle R₄₅-CO-, dans lequel R₄₅ est un radical alkyle en C₁-C₄ éventuellement substitué;
■ R₅ et R₆ formant éventuellement avec l'atome d'azote auquel ils sont rattachés, un hétérocycle renfermant 1 à 3 hétéroatomes choisis parmi N, O, S, de préférence N, comprenant de 5 à 7 chaînons, saturé ou insaturé, aromatique ou non, éventuellement substitué ;
■ R₅ et R₆, indépendamment l'un de l'autre peuvent former avec l'atome de carbone du cycle aromatique adjacent à celui auquel ―N R₅R₆, est rattaché, un hétérocycle saturé à 5 ou 6 chaînons ;
■ Ph représentant un radical phényle éventuellement substitué ;
R" représente
- un atome d'hydrogène,
- un radical alkyle en C₁-C₁₆, éventuellement substitué, éventuellement interrompu par un ou plusieurs hétéroatomes ou par un ou plusieurs groupements comprenant au moins un hétéroatome, de préférence choisi parmi l'oxygène, l'azote, le soufre, tels que -CO-, - SO₂- ou leurs combinaisons ;
- un radical aryle ou arylalkyle en C₆-C₃₀, tel que phényle, benzyle, la partie aryle étant éventuellement substituée, de préférence, par un ou plusieurs groupements identiques ou différents de préférence choisis parmi par un atome de chlore, un groupement amino, un groupement hydroxyle, un groupement alcoxy en C₁-C₂, un groupement amino mono ou disubstitué par deux radicaux alkyle identiques ou différents éventuellement porteurs d'au moins un groupement hydroxyle ;
   R"' représente
- un atome d'hydrogène,
- un radical alkyle en C₁-C₁₆, éventuellement substitué, éventuellement interrompu par un ou plusieurs hétéroatomes ou par un ou plusieurs groupements comprenant au moins un hétéroatome, de préférence choisi parmi l'oxygène, l'azote, le soufre, tels que -CO-, - SO₂- ou leurs combinaisons ;
- un radical aryle ou arylalkyle en C₆-C₃₀, tel que phényle, benzyle, la partie aryle étant éventuellement substituée, de préférence, par un ou plusieurs groupements identiques ou différents, de préférence, choisis parmi par un atome de chlore, un groupement amino, un groupement hydroxyle, un groupement alcoxy en C₁-C₂, un groupement amino mono ou disubstitué par deux radicaux alkyle identiques ou différents éventuellement porteurs d'au moins un groupement hydroxyle ;

R"" représente un atome d'hydrogène, un atome d'halogène choisi parmi le brome, le chlore, le fluor de préférence le chlore et le fluor, un groupement -OR₇. la liaison a* se situant :
- soit sur l'un des atomes d'azote quaternisé ou non des formules (IIIa) ou (IIIb),
- soit sur l'un des atomes de carbone des hétérocycles des formules (IIIa) ou (IIIb),
- soit sur l'un des atomes de carbone du cycle aromatique Ar,
- soit sur l'atome de carbone portant R" ou R''',
- soit sur l'atome d'azote portant les radicaux R₅, R₆,
- soit sur l'atome d'oxygène portant R₇,
   auquel cas le radical R₁, R₂, R₃, R", R"', R₅, R₆ ou R₇ concerné est remplacé par une liaison simple reliant L₁ à COL ;
   dans le cas ou R' représente le radical COL, L₂ est identique à L₁ et relié de la même manière à R' que L₁ à COL ;
   les cycles aromatiques des chromophores anthraquinoniques des formules la et Ib peuvent éventuellement être substitués ;
   l'électroneutralité des composés étant assurée par un ou plusieurs anions An-, identiques ou non, cosmétiquement acceptables.

De préférence, les groupements R représentent un atome d'hydrogène, un radical alkyle en C₁-C₂.

De manière préférée, R' représente :
- un atome d'hydrogène,
- une chaîne hydrocarbonée en C₁-C₆ linéaire ou ramifiée, éventuellement substituée, pouvant éventuellement être interrompue ou terminée par un ou plusieurs groupements de type : amino, mono ou di-alkylamino éventuellement substitué, ou bien
- le radical COL.

R₁, indépendamment les uns des autres représentent avantageusement une chaîne hydrocarbonée en C₁-C₁₀, linéaire ou ramifiée, saturée ou insaturée, pouvant former un ou plusieurs cycles carbonés comportant de 5 ou 6 chaînons, éventuellement condensés avec le cycle aromatique, éventuellement substituée.

De préférence, R₂, indépendamment les uns des autres, représentent:
- une chaîne hydrocarbonée en C₁-C₁₀, linéaire ou ramifiée, saturée ou insaturée, pouvant former un ou plusieurs cycles carbonés comportant de 5 ou 6 chaînons, éventuellement substitué par un ou plusieurs groupements identiques ou différents de préférence choisis parmi hydroxyle, alcoxy en C₁-C₂;
- un groupement hydroxyle,
- un groupement alcoxy en C₁-C₄,
- un groupement alcoxycarbonyle R₁₁O-CO-, dans lequel R₁₁ représente un radical alkyle en C₁-C₂ , un radical alkylcarbonyloxy R₁₂CO-O-, dans lequel R₁₂ représente un radical alkyle en C₁-C₂ ;
- un groupement amino ; un groupement amino substitué par un ou deux radicaux alkyles en C₁-C₄, identiques ou différents, éventuellement porteurs d'au moins un groupement hydroxyle, les deux radicaux alkyles formant éventuellement avec l'atome d'azote auquel ils sont rattachés, un hétérocycle à 5 ou 6 chaînons éventuellement porteur d'un autre hétéroatome identique ou différent de l' azote, par exemple l'oxygène, le soufre
- un groupement alkylcarbonylamino R₁₃CO-NR₁₄-, dans lequel les radicaux R₁₃ et R₁₄ indépendamment l'un de l'autre, représentent un radical alkyle en C₁-C₂ ; un groupement carbamoyle (R₁₅)₂N-CO, dans lequel les radicaux R₁₅ indépendamment l'un de l'autre, identiques ou non, représentent un atome d'hydrogène, un radical alkyle en C₁-C₂ ; un groupement uréido (R₁₆)₂N-CO-NR₁₇-, dans lequel les radicaux R₁₆ et R₁₇, indépendamment l'un de l'autre, représentent un atome d'hydrogène, un radical alkyle en C₁-C₄ ;
- un groupement sulfonamide (R₁₈)₂N-SO₂-, dans lequel les radicaux R₁₈, indépendamment l'un de l'autre, représentent un atome d'hydrogène, un radical alkyle en C₁-C₄;
- un groupement guanidinium (R₂₁)₂N-C(=NH₂⁺)-NR₂₂-, dans lequel les radicaux R₂₁ et R₂₂, indépendamment les uns des autres, représentent un atome d'hydrogène, un radical alkyle en C₁-C₄;
- un groupement alkylsulfonyle R₂₃-SO₂-, dans lequel R₂₃ représente un radical alkyle en C₁-C₄ ;
- un groupement cyano ; un atome d'halogène, de préférence le chlore, le fluor
- un groupement phényle éventuellement substitué.

Les radicaux R₂ identiques ou non, indépendamment les uns des autres représentent avantageusement un radical méthyle, éthyle, 2-hydroxyéthyle, 2-méthoxyéthyle, méthylsulfonyle (CH₃SO₂-), méthylcarbonylamino (CH₃CONH-), hydroxyle, amino, méthylamino, diméthylamino, 2-hydroxyéthylamino, bis(2-hydroxyéthyl)amino, méthoxy, éthoxy, phényle.

Selon un mode de réalisation préféré, les radicaux de formules (IIIa) et (IIIb) portent deux radicaux R₂, alors ces radicaux R₂ forment avec les atomes de carbone auxquels ils sont rattachés, un cycle secondaire, aromatique à 6 chaînons, éventuellement substitué par un ou plusieurs groupements identiques ou différents choisis parmi hydroxyle, alkyle en C₁-C₄, alcoxy en C₁-C₄, amino, amino substitué par un ou deux radicaux alkyle en C₁-C₄ identiques ou différents et éventuellement porteurs d'au moins un groupement hydroxyle ou méthylcarbonylamino.

Plus particulièrement, les deux radicaux R₂ forment avec les atomes de carbone auxquels ils sont rattachés, un cycle secondaire, aromatique à 6 chaînons, éventuellement substitué par un ou plusieurs hydroxyle, méthoxy, éthoxy, amino, 2-hydroxyéthylamino, diméthylamino, bis-(-2-hydroxyéthyl)amino.

De préférence, m représente un entier compris entre 0 et 2.

De préférence, e est compris entre 0 et 2, et plus particulièrement e est égal à 0.

Selon un mode de réalisation préféré, les radicaux R₃ identiques ou non, représentent:
- un radical alkyle en C₁-C₁₆, éventuellement substitué
- un groupement hydroxyle,
- un groupement alcoxy en C₁-C₂ ; un groupement (poly)hydroxyalcoxy en C₂-C₄ ;
- un groupement alcoxycarbonyle R₃₁O-CO-, dans lequel R₃₁ représente un radical alkyle en C₁-C₄ ; un radical alkylcarbonyloxy R₃₂CO-O-, dans lequel R₃₂ représente un radical alkyle en C₁-C₄ ;
- un radical alkylcarbonyle R₃₃-CO-, dans lequel R₃₃ représente un radical alkyle en C₁-C₄ ;
- un groupement amino ; un groupement amino substitué par un ou deux radicaux alkyle en C₁-C₄, identiques ou différents, éventuellement porteurs d'au moins un groupement hydroxyle ; les deux radicaux alkyle formant éventuellement avec l'atome d'azote auquel ils sont rattachés, un hétérocycle renfermant 1 à 3 hétéroatomes, de préférence 1 à 2 hétéroatomes, choisis parmi N, O, S, de préférence N, comprenant de 5 à 7 chaînons, saturé ou insaturé, aromatique ou non, éventuellement substitué ;
- un groupement alkylcarbonylamino R₃₄CO-NR₃₅-, dans lequel le radical R₃₄ représente un radical alkyle en C₁-C₄ et le radical R₃₅ représente un atome d'hydrogène ou un radical alkyle en C₁-C₄ ;
- un groupement aminocarbonyle (R₃₆)₂N-CO-, dans lequel les radicaux R₃₆ indépendamment l'un de l'autre, représentent un atome d'hydrogène, un radical alkyle en C₁-C₄ ;
- un groupement uréido N(R₃₇)₂-CO-NR₃₈-, dans lequel les radicaux R₃₇ et R₃₈, indépendamment l'un de l'autre, représentent un atome d'hydrogène, un radical alkyle en C₁-C₄ ;
- un groupement aminosulfonyle (R₃₉)₂N-SO₂-, dans lequel les radicaux R₃₉ indépendamment l'un de l'autre, représentent un atome d'hydrogène, un radical alkyle en C₁-C₄ ;
- un groupement alkylsulfonylamino R₄₀SO₂-NR₄₁-, dans lequel les radicaux R₄₀ et R₄₁, indépendamment l'un de l'autre, représentent un atome d'hydrogène, un radical alkyle en C₁-C₄ ;
- un groupement thiol HS- ;
- un groupement alkylthio R₄₂S-, dans lequel le radical R₄₂ représente un radical alkyle en C₁-C₄ ;
- un groupement alkylsulfonyle R₄₄-SO₂-, dans lequel R₄₄ représente un radical alkyle en C₁-C₄ ;
- un groupement cyano ;
- un atome d'halogène tel que le chlore, le fluor.

Plus particulièrement, les radicaux R₃, identiques ou différents, indépendamment les uns des autres, représentent :
- un radical alkyle en C₁-C₄ éventuellement substitué par un ou plusieurs radicaux identiques ou différents choisis parmi les radicaux hydroxyle, alkylcarbonylamino en C₁-C₂, amino substitué par deux radicaux alkyles en C₁-C₂ identiques ou différents éventuellement porteurs d'un ou plusieurs groupements identiques ou différents choisis parmi hydroxyle ou alcoxy en C₁-C₂ ces deux radicaux alkyle peuvent éventuellement former avec l'atome d'azote auquel ils sont rattachés, un hétérocycle à 5 ou 6 chaînons, saturé ou non, éventuellement aromatique, choisi de préférence parmi la pyrrolidine, la pipérazine, l'homopipérazine, le pyrrole, l'imidazole, le pyrazole ;
- un radical alkyle en C₁-C₄ éventuellement interrompu par un ou plusieurs hétéroatomes ou par un ou plusieurs groupements comprenant au moins un hétéroatome, de préférence choisi parmi l'oxygène, l'azote, le soufre, tels que -CO-, -SO₂- ou leurs combinaisons
- un radical hydroxyalcoxy en C₂-C₄ ;
- un halogène choisi parmi le chlore ou le fluor ;
- un radical amino ;
- un radical amino substitué par un ou deux radicaux alkyles en C₁-C₂ , identiques ou différents éventuellement porteurs d'au moins un groupement hydroxyle;
- un radical méthylcarbonylamino ;
- un radical méthylsulfonylamino ;
- un radical hydroxyle ;
- un radical alcoxy en C₁-C₂ ;
- un radical méthylsulfonyle.

Encore plus particulièrement, les radicaux R₃, identiques ou différents, indépendamment les uns des autres, représentent:
- un radical méthyle, éthyle, propyle, 2-hydroxyéthyle ;
- un radical méthoxy, éthoxy ;
- un radical 2-hydroxyéthyloxy, 3-hydroxypropyloxy ;
- un radical 2-méthoxyéthyle ;
- un radical méthylsulfonylamino ;
- un radical amino, méthylamino, diméthylamino, 2-hydroxyéthylamino.
- un radical méthylcarbonylamino ;
- un radical hydroxyle ;
- un atome de chlore ;
- un radical méthylsulfonyle,
- Avantageusement, lorsque le coefficient n est supérieur ou égal à 2, alors deux radicaux R₃ adjacents forment avec les atomes de carbone auxquels ils sont rattachés, un cycle secondaire aromatique à 6 chaînons, éventuellement substitué par un ou plusieurs groupements identiques ou différents choisis parmi les groupements hydroxyle, alkyle en C₁-C₄, alcoxy en C₁-C₄, (poly)hydroxyalcoxy en C₂-C₄, alkylcarbonylamino en C₁-C₄, amino, amino substitué par un ou deux radicaux identiques ou différents, alkyle en C₁-C₄ éventuellement porteurs d'au moins un groupement hydroxyle, aryle.

De préférence, deux radicaux R₃ adjacents forment avec les atomes de carbone auxquels ils sont rattachés, un cycle secondaire aromatique à 6 chaînons, éventuellement substitué par un ou plusieurs groupements choisis parmi les groupements hydroxyle, méthoxy, éthoxy, 2-hydroxyethyloxy, amino, methylcarbonylamino, (di)2-hydroxyethylamino, -NH-Ph, -NH-Ph-NH₂, -NH-Ph-NHCOCH₃, -NH-Ph-OH, -NH-Ph-OCH₃.

De préférence, n représente un entier compris entre 0 et 2.

Selon un mode de réalisation particulier, R₄, et R₇ représentent:
- un atome d'hydrogène ;
- un radical alkyle en C₁-C₆ éventuellement substitué par un ou plusieurs groupements identiques ou différents de préférence choisis parmi hydroxyle, alcoxy en C₁-C₂;
- un radical aryle ou arylalkyle, tel que phényle, benzyle, la partie aryle étant éventuellement substituée par un ou plusieurs groupements identiques ou différents de préférence choisis parmi par un atome de chlore, un groupement amino, un groupement hydroxyle, un groupement alcoxy en C₁-C₂, un groupement amino mono ou disubstitué par deux radicaux alkyle identiques ou différents identiques ou différents éventuellement porteurs d'au moins un groupement hydroxyle ;

De manière encore plus préfère, les radicaux R₄ et R₇, représentent :
- un atome d'hydrogène ;
- un radical alkyle en C₁-C₃ éventuellement substitué, tel que méthyle, éthyle, 2-hydroxyéthyle, 2-méthoxyéthyle ;
- un radical phényle, éventuellement substitué par un ou plusieurs radicaux identiques ou différents choisis parmi les radicaux hydroxyle, alcoxy en C₁-C₂, amino, amino substitués par un ou plusieurs groupements alkyle en C₁-C₄ éventuellement porteurs d'au moins un groupement hydroxyle.

Plus particulièrement, les radicaux R₄ et R₇ représentent :
- un atome d'hydrogène ;
- un radical méthyle, éthyle, 2-hydroxyéthyle ;
- un radical phényle, éventuellement substitué par un ou plusieurs radicaux identiques ou différents choisi parmi hydroxyle, méthoxy, amino, (di)méthylamino , (di)(2-hydroxyéthyl)amino.

Selon un mode de réalisation particulier, R₅ et R₆, indépendamment l'un de l'autre, identiques ou non, représentent:
- un atome d'hydrogène ;
- un radical alkylcarbonyle R₄₅-CO-, dans lequel R₄₅ représente un radical alkyle en C₁-C₄ éventuellement substitué,
- un radical alkyle en C₁-C₆ éventuellement substitué par un ou plusieurs groupements identiques ou non, choisi parmi hydroxyle, alcoxy en C₁-C₂, amino, (di-)alkyle amino en C₁-C₄ ; le radical alkyle peut en outre être substitué par un ou plusieurs groupements identiques ou non, choisi parmi alkylsulfonyle en C₁-C₄ , alkylsulfinyle en C₁-C₄ , alkylcarbonyle en C₁-C₄,
- un radical aryle, ou arylalkyle, tel que phényle, benzyle, la partie aryle étant éventuellement substituée, de préférence par un atome de chlore, un groupement amino, un groupement hydroxyle, un groupement alcoxy en C₁-C₄ , un groupement amino mono- ou di-substitué par deux radicaux identiques ou différents, choisis parmi les radicaux alkyle en C₁-C₄ éventuellement porteurs d'au moins un groupement hydroxyle.

Plus particulièrement, les radicaux R₅ et R₆, identiques ou différents, indépendamment l'un de l'autre représentent :
- un atome d'hydrogène ;
- un radical méthylcarbonyle, éthylcarbonyle, propylcarbonyle ;
- un radical alkyle en C₁-C₃ éventuellement substitué, tel que méthyle, éthyle, 2-hydroxyéthyle, 2-méthoxyéthyle ;
- un radical phényle, éventuellement substitué par un ou plusieurs radicaux identiques ou non choisis parmi les radicaux hydroxyle, alcoxy en C₁-C₂, amino, amino substitués par un ou plusieurs groupements alkyle en C₁-C₄ éventuellement porteurs d'au moins un groupement hydroxyle.

Selon une variante de l'invention, les radicaux R₅ et R₆, identiques ou différents, indépendamment l'un de l'autre, représentent :
- un atome d'hydrogène ;
- un radical méthyle, éthyle, 2-hydroxyéthyle ;
- un radical méthylcarbonyle, éthylcarbonyle, propylcarbonyle ;
- un radical phényle, éventuellement substitué par un radical hydroxyle, méthoxy, amino, (di)méthylamino , (di)(2-hydroxyéthyl)amino.

Selon une autre variante de l'invention, les radicaux R₅ et R₆ forment ensemble avec l'atome d'azote auquel chacun est rattaché, un hétérocycle renfermant 1 à 2 hétéroatomes, choisis parmi N, O, S, de préférence N, comprenant de 5 à 7 chaînons, saturé ou insaturé, aromatique ou non, éventuellement substitué.

De préférence, l'hétérocycle comprenant de 5 à 7 chaînons représente un hétérocycle de type pipéridine, pipérazine, homopipérazine, pyrrole, imidazole, pyrazole éventuellement substitué par un ou plusieurs radicaux identiques ou non choisis parmi les radicaux hydroxyle, alcoxy en C₁-C₂, amino, amino substitués par un ou plusieurs groupements alkyle en C₁-C₄ éventuellement porteurs d'au moins un groupement hydroxyle.

De préférence, les radicaux R₅ et R₆ forment ensemble avec l'atome d'azote auquel chacun est rattaché, un hétérocycle comprenant de 5 à 7 chaînons choisi parmi les hétérocycles suivants : pipéridine, 2-(2-hydroxyéthylpipéridine), 4-(aminométhyl)pipéridine, 4-(2-hydroxy éthyl)pipéridine, 4-(diméthylamino)pipéridine, pipérazine, 1-méthylpipérazine, 1-(2-hydroxyéthyl)pipérazine, 1-(2-aminoéthyl)pipérazine, 1-hydroxyéthyléthoxy pipérazine, homopipérazine, 1-méthyl-1,4-perhydrodiazépine, pyrrole, 1,4-diméthylpyrrole, 1-méthyl-4-éthylpyrrole, 1-méthyl-4-propylpyrrole.

De manière préférée, les radicaux R₅ et R₆ peuvent former, avec l'atome de carbone du cycle aromatique éventuellement substitué par un hydroxyle et adjacent à celui auquel -NR₅R₆, est rattaché, un hétérocycle saturé à 5 ou 6 chaînons ou des hétérocycles condensés saturés à 5 ou 6 chaînons.

De préférence, lorsque W représente -NR₅R₆, et que la liaison b* se trouve en para de -NR₅R₆, alors le groupement -NR₅R₆, avec le noyau aromatique éventuellement substitué par un hydroxyle répond à l'une des formules suivantes: 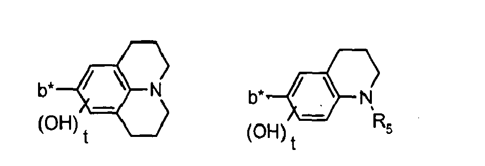 dans laquelles
t est égal à 0 ou 1,
R₅ a la même signification que précisée ci-dessus
b* a la même signification que précisé ci-dessus.

Selon une variante de l'invention, R" représente un atome d'hydrogène ou un radical alkyle en C₁-C₁₀, éventuellement substitué.

De préférence, R"' représente un atome d'hydrogène ou un radical alkyle en C₁-C₁₀, éventuellement substitué.

De manière préférée, la liaison a* se situant :
- soit sur l'un des atomes d'azote quaternisé ou non des formules (IIIa) ou (IIIb),
- soit sur l'atome d'azote portant les radicaux R₅ et R₆.
- soit sur l'un des atomes du groupement R₅, R₆ ou R₇,

Selon un premier mode de réalisation, l'un au moins des bras de liaison L₁ et L₂ représente un bras de liaison non ionique.

De préférence, L₁, L₂, indépendamment l'un de l'autre, sont choisis parmi:
- un radical alkylène en C₁-C₂₀, éventuellement substitué, éventuellement interrompu par un (hétéro)cycle saturé ou insaturé, aromatique ou non, comprenant de 3 à 7 chaînons, éventuellement substitué, éventuellement condensé ; le dit radical alkylène étant éventuellement interrompu par un ou plusieurs hétéroatomes ou groupements comprenant au moins un hétéroatome de préférence l'oxygène, l'azote, le soufre, tels que -CO-,
   - SO₂- ou leurs combinaisons ; le bras de liaison L1 et/ou L2 ne comprenant pas de fonction azo, nitro, nitroso, peroxo, L₂ représente une liaison covalente, lorsque R' est différent de COL.

L₁, L₂, indépendamment l'un de l'autre, représentent avantageusement un radical alkylène choisi parmi méthylène, éthylène, propylène linéaire ou ramifié, butylène linéaire ou ramifié, pentylène linéaire ou ramifié, hexylène linéaire ou ramifié, éventuellement substitués et/ou interrompus par un ou plusieurs hétéroatomes ou groupements comprenant au moins un hétéroatome de préférence l'oxygène, l'azote, le soufre, tels que -CO-, -SO₂- ou leurs combinaisons ; le bras de liaison L1 et/ou L2 ne comprenant pas de fonction azo, nitro, nitroso et peroxo.

Plus particulièrement, L₁, L₂, indépendamment l'un de l'autre, représentent un radical alkylène substitué par des substituants, identiques ou différents, choisis parmi l'hydroxyle, l'alcoxy en C₁-C₂, le dialkylamino en C₁-C₂, l'alkyl(C₁-C₄)carbonyle, l'alkyl(C₁-C₄)sulfonyle.

En particulier, le cycle ou l'hétérocycle, saturé ou insaturé, aromatique ou non, pouvant interrompre le radical alkylène du bras de liaison L1 et/ou L2 est choisi parmi le phénylène, naphtylène, phénanthrylène, triazinyle, pyrimidinyle, pyridinyle, pyridazinyle, quinoxalinyle, cyclohexyle.

L₁ et/ou L₂, indépendamment l'un de l'autre, sont avantageusement choisis parmi les radicaux suivants : 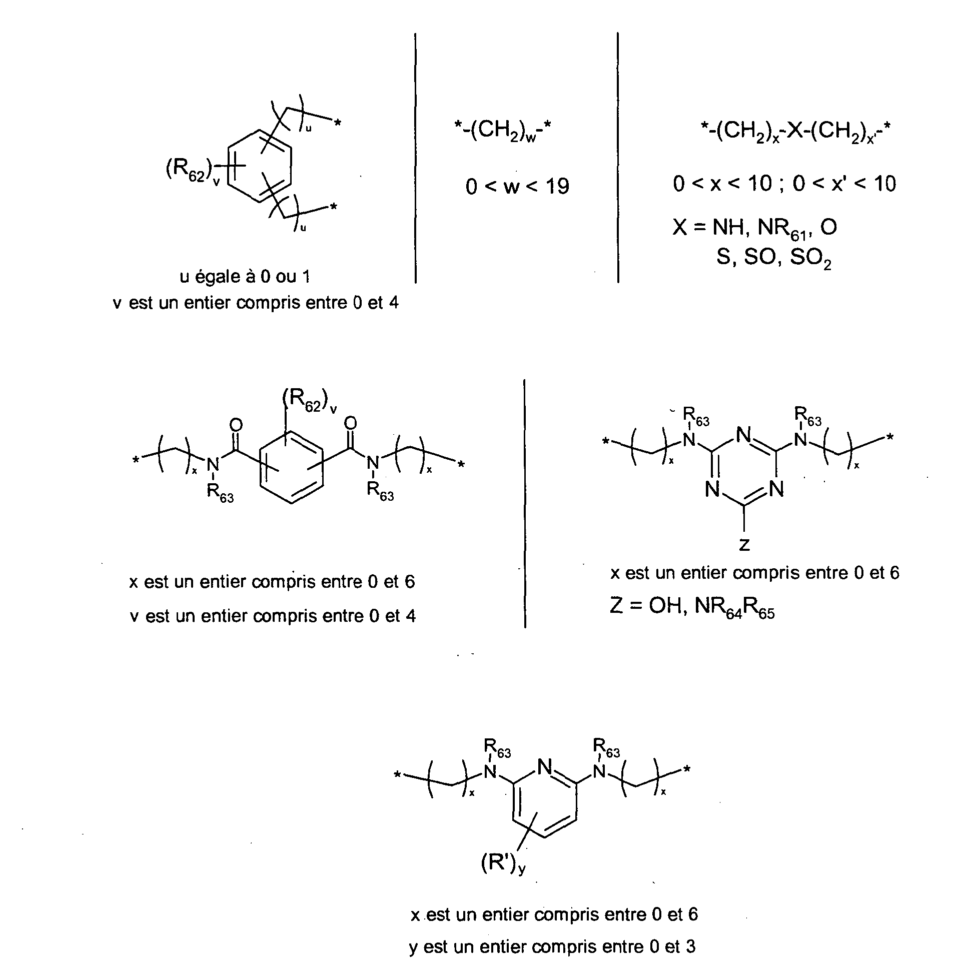 dans lesquelles :
- R₆₂ et R' ont la même définition que R₃ ;
- R₆₃ identiques, représentent un hydrogène, un radical alkyle en C₁-C₄ ;
- R₆₄ et R₆₅ représentent, indépendamment l'un de l'autre, un atome d'hydrogène, un radical alkyle en C₁-C₈ éventuellement substitué par un ou plusieurs radicaux identiques ou différents choisis parmi hydroxyle, alcoxy en C₁-C₂, (poly)hydroxyalcoxy en C₂-C₄, amino, (di-) alkylamino en C₁-C₂ ou aryle éventuellement substitué,
- * représentent les extrémités des bras de liaison L₁ et/ou L₂,
- R₆₁ représente un radical alkyle en C₁-C₄ éventuellement substitué par un ou plusieurs radicaux identiques ou différents choisis parmi hydroxyle, alcoxy en C₁-C₂, (poly)hydroxyalcoxy en C₂-C₄, amino, (di-) alkylamino en C₁-C₂ ou aryle éventuellement substitué.

Selon un deuxième mode de réalisation de l'invention, l'un au moins des bras de liaison L₁ et L₂ représente un bras de liaison portant au moins une charge cationique.

De préférence, L₁, L₂, indépendamment l'un de l'autre, représentent un radical alkylène en C₂-C₄₀, portant au moins une charge cationique, éventuellement substitué et/ou éventuellement interrompu par un ou plusieurs (hétéro)cycle saturé ou non, aromatique ou non, identique ou différent, comprenant de 5 à 7 chaînons et/ou éventuellement interrompu par un ou plusieurs hétéroatomes ou groupements comprenant au moins un hétéroatome ou leurs combinaisons, comme par exemple l'oxygène, l'azote, le soufre, un groupement -CO-, -SO₂- ou leurs combinaisons ; le bras de liaison L₁ et/ou L₂ ne comprenant pas de fonction azo, nitro, nitroso et peroxo.

Avantageusement, la ou les charges cationiques sont portées par un ou plusieurs atome d'azote quaternisé, éventuellement engagé dans un hétérocycle comprenant de 5 à 6 chaînons, saturé ou non, substitué ou non, et comprenant éventuellement un ou plusieurs autres hétéroatomes choisis parmi l'oxygène, le soufre et de préférence l'azote.

De manière encore préférée, le bras de liaison L₁ et/ou L₂ cationique représente un radical alkyle en C₂-C₂₀ :
- interrompu par au moins un groupement correspondant aux formules suivantes : 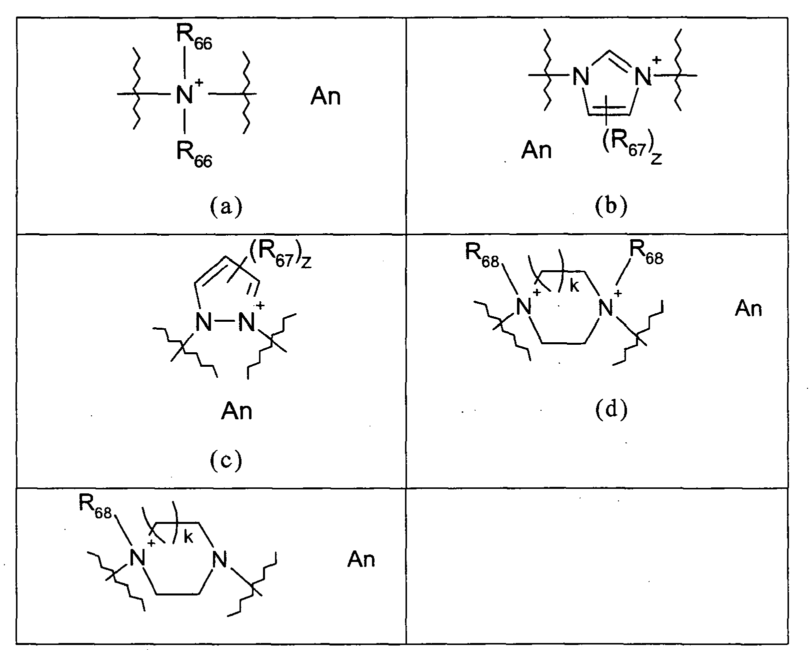
dans lesquelles :
- R₆₆ et R₆₈ indépendamment l'un de l'autre, représentent un radical alkyle en C₁-C₈ ; un radical monohydroxyalkyle en C₁-C₆ ; un radical polyhydroxyalkyle en C₂-C₆ ; un radical alcoxy(C₁-C₆)alkyle en C₁-C₆; un radical aryle, tel que phényle, éventuellement substitué; un radical arylalkyle, tel que benzyle, éventuellement substitué; un radical aminoalkyle en C₁-C₆; un radical aminoalkyle en C₁-C₆ dont l'amine est substituée par un ou deux radicaux alkyle C₁-C₄ identiques ou différents, un radical alkyl(C₁-C₆)sulfonyle.
- deux radicaux R₆₆ peuvent former ensemble, avec l'atome d'azote auxquels ils sont rattachés, un cycle saturé ou insaturé, éventuellement substitué, à 5, 6 ou 7 chaînons.
- deux radicaux R₆₈ peuvent former ensemble, avec l'atome d'azote auxquels ils sont rattachés, un cycle saturé à 6 chaînons.
- R₆₇, identiques ou différents, représentent un atome d'halogène choisi parmi le brome, le chlore ou le fluor, un radical alkyle en C₁-C₆, un radical monohydroxyalkyle en C₁-C₆, un radical polyhydroxyalkyle en C₂-C₆, un radical alcoxy en C₁-C₆, un radical (di-)alkylamino en C₁-C₄, un radical hydroxycarbonyle, un radical alkylcarbonyle en C₁-C₆, un radical thioalkyle en C₁-C₆, un radical alkyl(C₁-C₆)sulfonyle, un radical benzyle éventuellement substitué, un radical phényle éventuellement substitué par un ou plusieurs radicaux choisis parmi les radicaux méthyle, hydroxyle, amino, méthoxy.
- An est un anion ou un mélange d'anions, organiques ou minéraux
- z est un nombre entier compris entre 1 et 3 ; si z inférieur à 3, alors les atomes de carbone non substitués portent un atome d'hydrogène.
- k est un entier égal à 1 ou 2 de préférence égal à 1.

En outre, L1 et/ou L2, indépendamment l'un de l'autre, peuvent éventuellement être interrompus par un ou plusieurs hétéroatomes ou groupements comprenant au moins un hétéroatome ou leurs combinaisons, comme par exemple l'oxygène, l'azote, le soufre, un groupement -CO-, un groupement -SO₂- ; à la condition qu'il n'y ait pas de groupe ou liaison nitro, nitroso, peroxo dans le bras de liaison L₁ et/ou L₂.

Par ailleurs, L1 et/ou L2, indépendamment l'un de l'autre, peuvent être éventuellement substitués par un ou plusieurs radicaux choisis parmi les radicaux hydroxyle, alcoxy en C₁-C₂, (poly)-hydroxyalcoxy en C₂-C₄, amino substitué par un ou plusieurs groupements alkyle linéaires ou ramifiés en C₁-C₂ éventuellement porteurs d'au moins un groupement hydroxyle.

De préférence, les radicaux R₆₆ et R₆₈ des formules (a) et (d), séparément, sont choisis parmi un radical alkyle en C₁-C₆, un radical monohydroxyalkyle en C₁-C₄, un radical polyhydroxyalkyle en C₂-C₄, un radical alcoxy(C₁-C₆)alkyle en C₂-C₄, un radical diméthylaminoalkyle en C₂-C₆.

De préférence, les radicaux R₆₆ et R₆₈ séparément représentent un radical méthyle, éthyle, 2-hydroxyéthyle.

De préférence, le radical R₆₇ des formules (b) et (c) représente un atome d'halogène choisi parmi le chlore et le fluor, un radical alkyle en C₁-C₆, un radical monohydroxylalkyle en C₁-C₄, un radical alcoxy en C₁-C₄, un radical hydroxycarbonyle, un radical thioalkyle en C₁-C₆, un radical amino disubstitué par un radical alkyle en C₁-C₄.

Plus particulièrement, le radical R₆₇ des formules (b) et (c) représente un atome de chlore, un méthyle, un éthyle, un 2-hydroxyéthyle, un methoxy, un hydroxycarbonyle, un dimethylamino.

En particulier, z des formules (b) et (c) est égal à 0.

Les colorants mixtes selon l'invention plus particulièrement préférés utilisables dans les compositions de coloration capillaire sont :
- Anthraquinones reliées à deux chromophores azoïques en série azo-imidazolium : 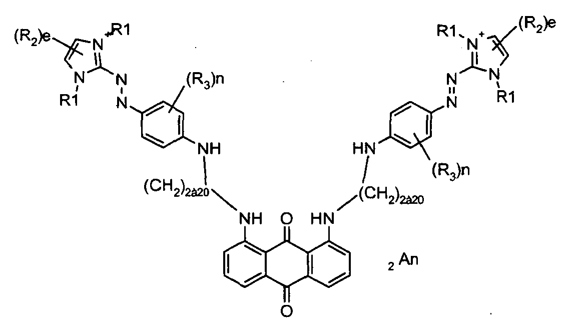
- Anthraquinones reliées à un chromophore azoïque en série azo-imidazolium : 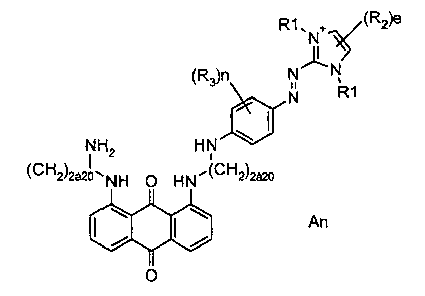
- Anthraquinones reliées à deux chromophores azoïques en série azo-imidazolium : 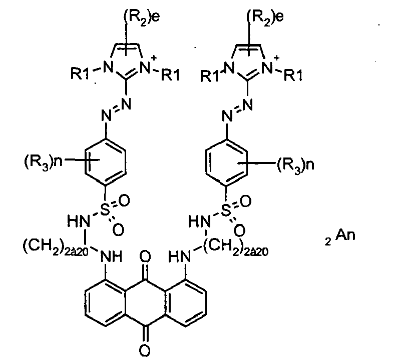
- Anthraquinones reliées à deux chromophores azoïques en série 3-azopyridinium : 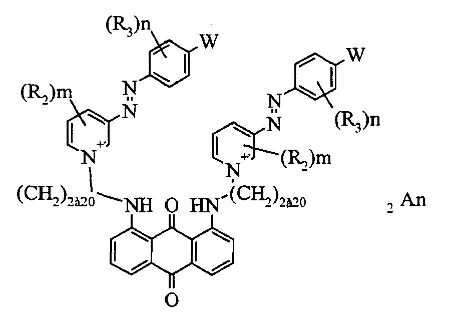
- Anthraquinones reliées à deux chromophores hydrazones en série 4-pyridinium : 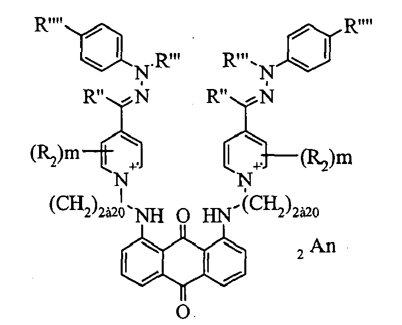
- Anthraquinones reliées à deux chromophores hydrazones en série 4-pyridinium - variante pour laquelle le bras de liaison est cationique 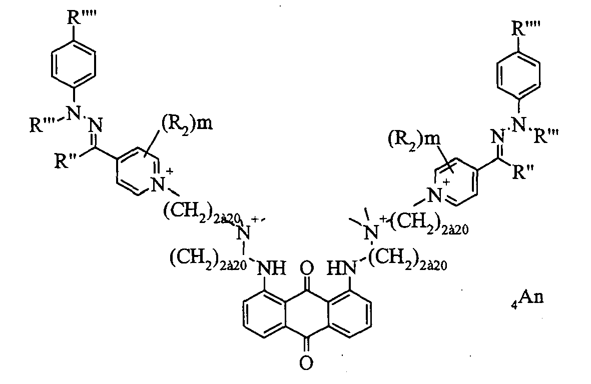
- Anthraquinones reliées à un chromophore hydrazone en série 4-pyridinium - variante pour laquelle le bras de liaison est cationique 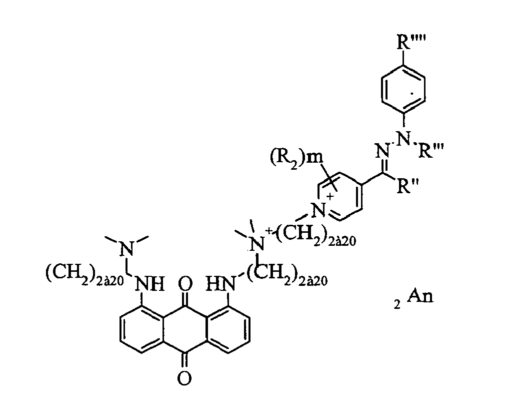
- Anthraquinones reliées à un chromophore hydrazone en série 4-quinolinium - variante pour laquelle le bras de liaison est cationique : 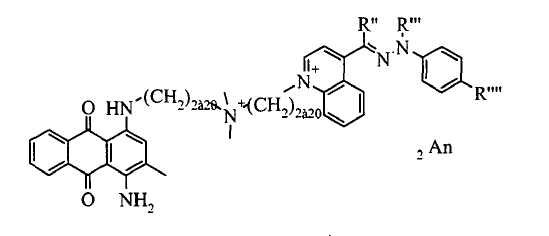
- Anthraquinones reliées à un chromophore hydrazone en série 4-pyridinium - variante pour laquelle le bras de liaison est cationique : 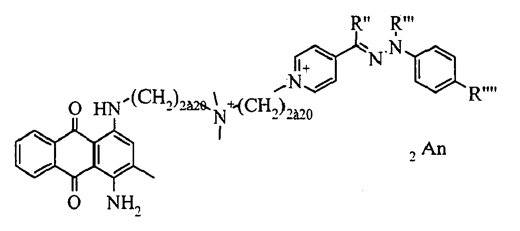
- Anthraquinones reliées à un chromophore azoïque en série azo-imidazolium : 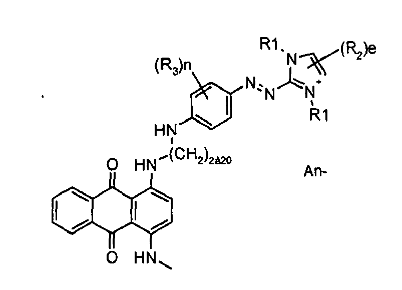
ou l'un de leurs sels ou solvates physiologiquement acceptables.

De préférence, les colorants mixtes selon l'invention utilisables dans les compositions de coloration capillaire sont
Anthraquinones reliées à deux chromophores azoïques en série azo-imidazolium : 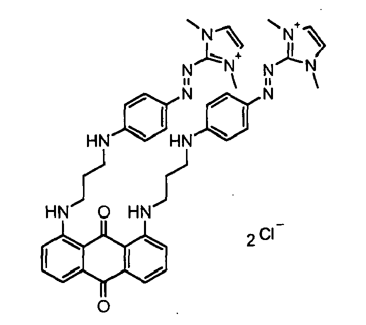 Anthraquinones reliées à un chromophore azoïque en série azo-imidazolium : 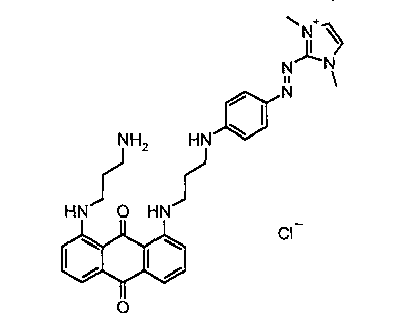 Anthraquinones reliées à deux chromophores azoïques en série azo-imidazolium : 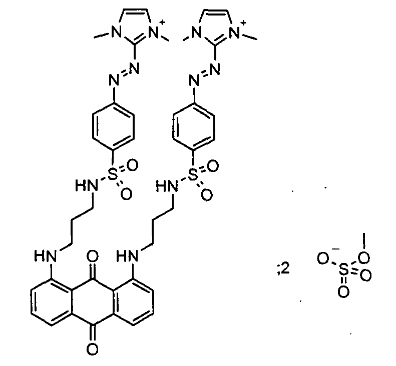 Anthraquinones reliées à deux chromophores azoïques en série 3-azopyridinium : 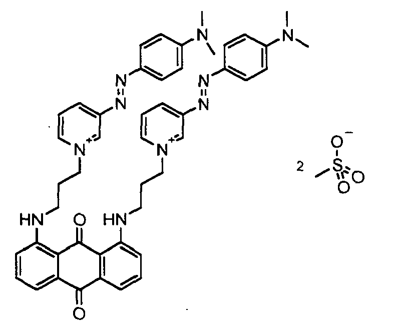 Anthraquinones reliées à deux chromophores hydrazones en série 4-pyridinium : 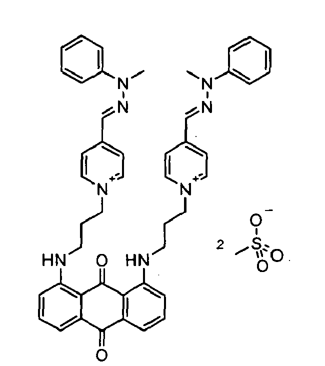 Anthraquinones reliées à deux chromophores hydrazones en série 4-pyridinium - variante pour laquelle le bras de liaison est cationique 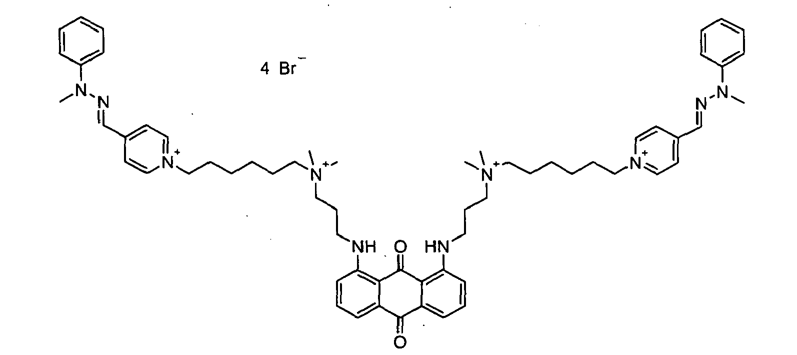 Anthraquinones reliées à un chromophore hydrazone en série 4-pyridinium ― variante pour laquelle le bras de liaison est cationique 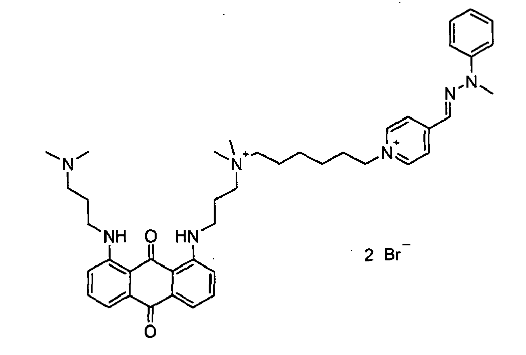 Anthraquinones reliées à un chromophore hydrazone en série 4-quinolinium - variante pour laquelle le bras de liaison est cationique : 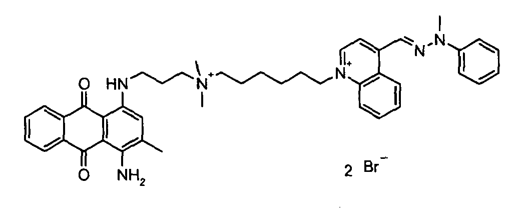 Anthraquinones reliées à un chromophore hydrazone en série 4-pyridinium - variante pour laquelle le bras de liaison est cationique : 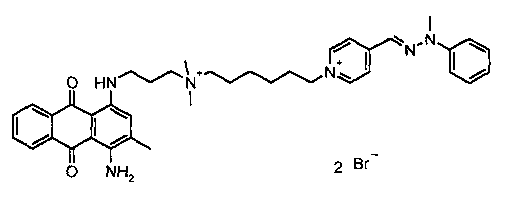 Anthraquinones reliées à un chromophore azoïque en série azo-imidazolium :

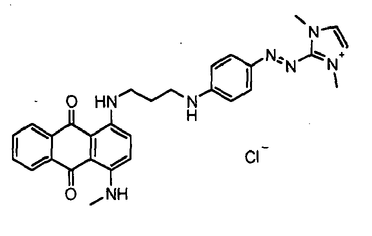

De très nombreux procédés de préparation généralement connus de l'homme de l'art permettent d'obtenir les produits suivant l'invention. On se réfèrera utilement aux ouvrages généraux de synthèse organique, tel que Advanced Organic Synthesis 5th Ed M. Smith and J. March John Wiley & Sons Ed, 2001, Color Chemistry, 3rd Ed Wiley VCH Ed 2003, H Zollinger, Color Chemistry, Wiley VCH Ed 2003 et The Chemistry of synthetic dyes, Academic Press, London, vol II, 1952.

Plusieurs réactions génériques peuvent être notamment utilisées pour l'élaboration de diverses molécules répondant à l'invention, parmi lesquelles, et sans exclusive :
1) substitutions nucléophiles aromatiques sur des molécules de la famille des anthraquinones
   1.1) substitutions nucléophiles par remplacement d'un groupe partant
      Les réactions sont du type : 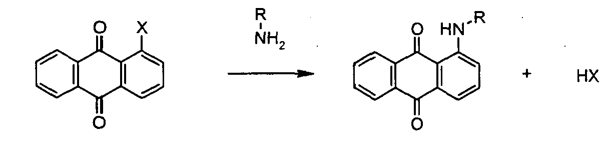 Où X représente de préférence un chlorure ou un bromure (la substitution d'un mésylate, d'un tosylate ou d'un fluorure étant également possible).
      Les conditions de réactions usuelles sont une mise en présence de l'amine, éventuellement introduite en excès du rapport stoechiométrique dans un rapport de 1 pour 1 à 100 pour 1 et de l'haloanthraquinone, sans solvant ou en présence d'un solvant, de préférence polaire, tel que par exemple, la DMF(N,N-diméthylformamide), la DMPU (1,3-diméthyle-3,4,5,6-tétrahydro-2(1H)-pyrimidinone), la NMP(N-méthylpyrrolidone), le cas échéant en présence d'une base autre qu'une amine primaire ou secondaire, telle que par exemple la triéthylamine, la pyridine, l'hydroxyde de calcium, mais aussi en présence de catalyseurs tels que les sels de métaux de transition et tout particulièrement les sels de cuivre (I). Les conditions de réactions sont une durée comprise entre 5 minutes et 5 jours, de préférence entre 1 heure et 24 heures, à une température comprise entre la température ambiante, et 200°C, de préférence entre 60°C et 130°C.
      En référence à ce type de réaction, on peut citer Dyes and Pigments, 1981, p125-132.
      Cette réaction extrêmement utile dans le cadre de l'invention sera détaillée plus avant dans trois exemples de préparation de produits.
   1.2) substitutions nucléophiles par déplacement d'hydroxyle
      Le déplacement par une amine de l'un ou des deux groupements hydroxyle de l'alizarine est connu et permet d'obtenir à moindre coût des anthraquinones bis-aminées en 1,4, particulièrement utiles pour l'objet de l'invention. On trouvera les modalités de réaction notamment dans The Chemistry of synthetic dyes, Academic Press, London, vol II, 1952.
      Le schéma général de réaction est le suivant : 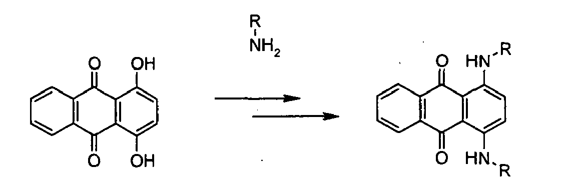 Les deux réactions précitées, sont, sans exclusive, particulièrement adaptées pour la préparation des produits suivant l'invention, qu'il s'agisse des produits finis (auquel cas R comportera le chromophore de la famille des azoïques cationiques ou des hydrazones cationiques ou le groupement R' ou R'-L₂), ou des intermédiaires de synthèse, précurseurs d'intérêt pour l'un, mais souvent pour plusieurs produits suivant l'invention.
2) réactions de substitutions nucléophiles aromatiques sur un colorant azoïque cationique 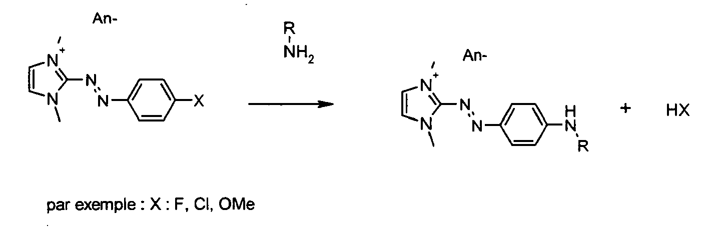 Cette réaction est particulièrement utile dans le cadre de l'invention puisqu'elle permet de connecter de façon simple un chromophore azoïque à un bras de liaison. Elle sera détaillée plus avant dans le cas d'un exemple particulier d'illustration. Elle s'effectue en solution, de préférence dans un solvant polaire, tel qu'un alcool ou un formamide, à une température comprise entre 0°C et 160°C, de préférence entre 40°C et 120°C, la réaction s'effectuant sur une durée comprise entre 5 min. et 5 jours, de préférence entre 1 et 48h.
   En référence à ce type de réaction, on peut citer Dyes & Pigment, 31(3), 1996, 219-224 et le brevet US 5,708,151.
3) réaction de diazotation pour la préparation d'un chromophore azoïque 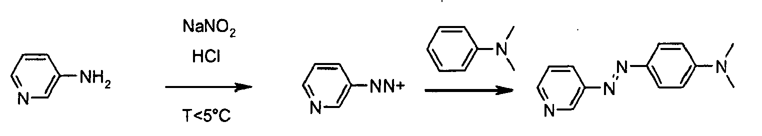 Cette réaction est une réaction classique utilisée pour la préparation de composé azoïques cationiques, une variante consiste à utiliser les aminopyridine-oxydes comme forme protégées des aminopyridines, et est d'une très grande utilité pour la préparation de composés azoïques en série 2-azopyridinium, une deuxième variante consiste à former intermédiairement un sel de diazonium à partir d'une aniline primaire puis à condenser sur ce sel un hétérocycle, comme l'illustre le schéma suivant : 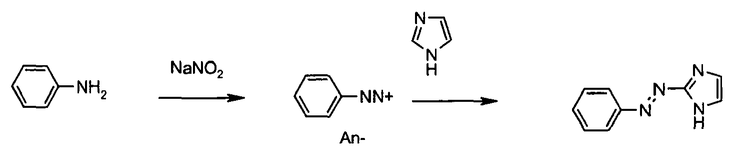 Cette réaction est également d'un très grand intérêt, comme il le sera explicité plus avant pour la description d'un exemple suivant l'invention. Elle est abondamment décrite dans les ouvrages déjà mentionnés et dans de nombreux brevets décrivant les colorants azoïques cationiques
4) réaction de substitution nucléophile aromatique par une hydrazine, suivie d'oxydation pour la préparation d'un chromophore azoïque 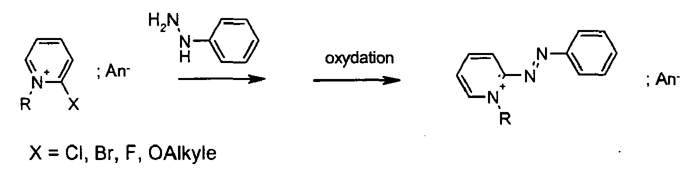 Il s'agit ici d'un procédé alternatif aux deux premiers, très utile dans le cas où les composés utilisés pour cette voie sont plus aisément accessibles ou bien dans le cas ou les sels de diazonium nécessaires aux procédés décrits précédemment sont instables et impropres à la préparation des produits désirés.
5) condensation d'une hydrazine sur un dérivé carbonylé pour la préparation d'un chromophore hydrazone 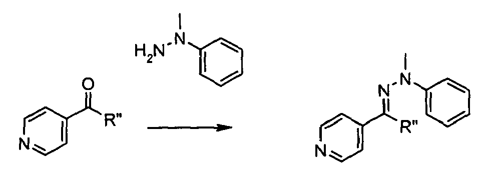 Cette réaction est particulièrement utile et bien connue pour la préparation de colorants de la famille des hydrazones, elle trouve toute son utilité dans l'invention pour la formation de ces chromophores, dans le cas, général, où ceux-ci ne sont pas disponibles dans le commerce. Cette réaction s'effectue avec de très bons rendements dans les solvants polaires tels que les alcools, en présence de préférence d'un réactif acide, tel qu'un acide carboxylique. La réaction s'effectue à une température comprise entre 0°C et 100°C, de préférence entre 10°C et 80°C. La durée de réaction est comprise entre 1 min. et 48h, de préférence entre 5 min et 24h.
   En référence à ce type de réaction, on peut citer le brevet WO 03/060015
6) Substitution nucléophile d'une amine sur un halogénure de sulfonyle 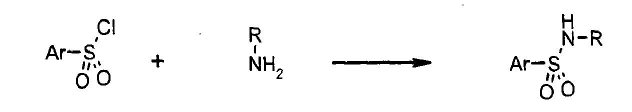 Cette réaction est d'un grand intérêt, car elle permet de lier ensemble deux groupements ; elle est mise à profit dans le cadre de l'invention pour relier les bras de liaison L₁, par exemple, à un chromophore ou à un précurseur de chromophore ; elle sera explicitée plus avant dans le cadre d'un exemple.
7) Alkylation d'un cycle hétéroaromatique par un halogénure d'alkyle ou par un ester sulfonique (dialkyle sulfate, alkyle sulfonate) 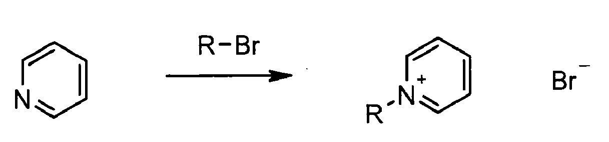 Cette réaction est d'une importance capitale pour l'invention ici présentée : elle permet notamment de procéder à la quaternisation des groupements azoïques ou hydrazones, et notamment d'utiliser les hétéroatomes des cycles A comme point d'ancrage pour les bras de liaison L₁. Elle sert aussi à procéder à la quaternisation de groupements L₁ dans le cas ou ceux-ci sont cationiques, enfin, elle permet d'introduire une fonction cationique supplémentaire, par exemple sur un groupement R'. En référence à ce type de réaction, on peut citer le brevet WO 03/060015.
   Les séquences de réactions permettant d'obtenir ces produits sont notamment illustrées par les schémas de synthèse ci-après. Elles sont plus amplement détaillées pour certains exemples de démonstration par la suite. 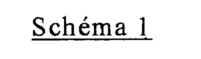 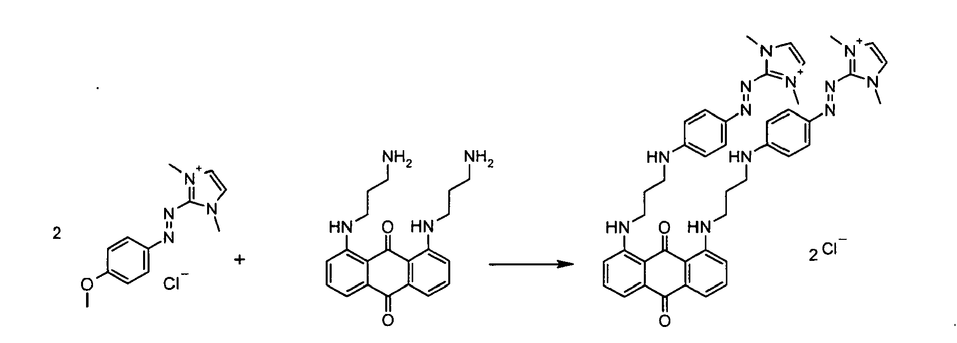 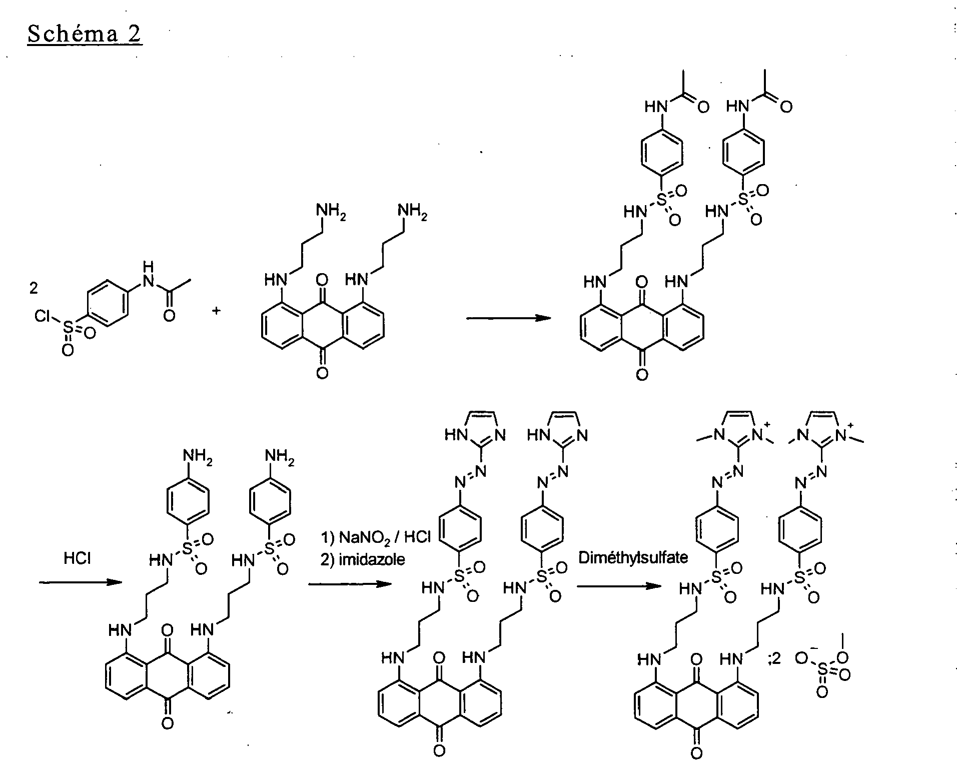 Cette stratégie de synthèse représente une alternative à la méthode décrite précédemment et particulièrement utile dans le cas où on ne dispose pas d'un groupement labile sur le colorant azoïque à greffer.

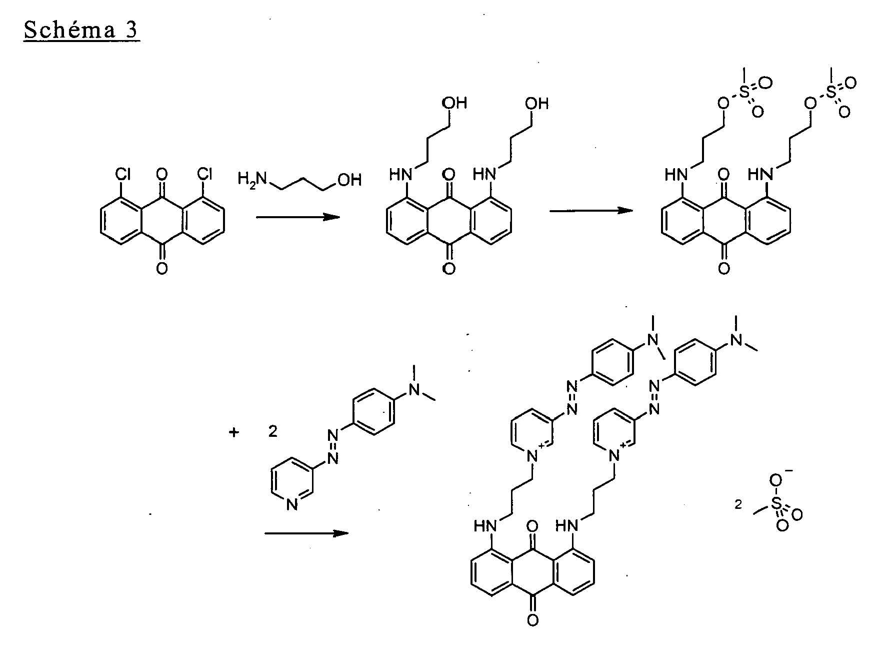

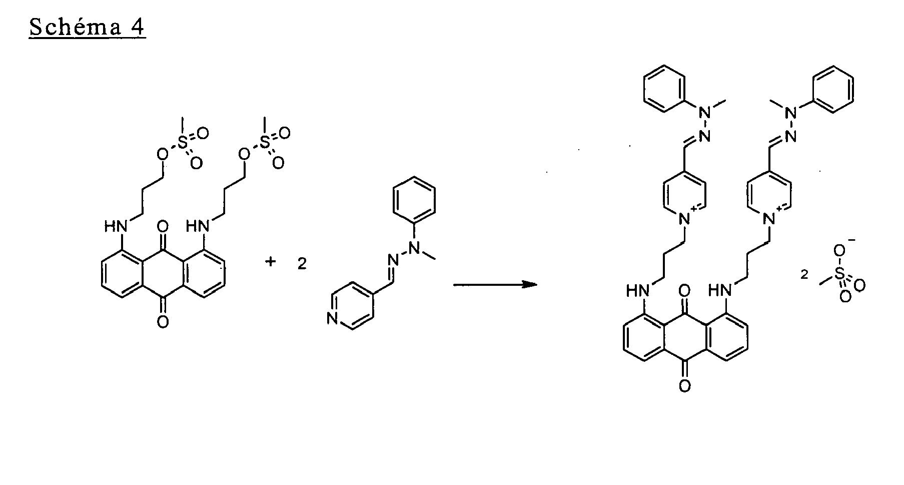

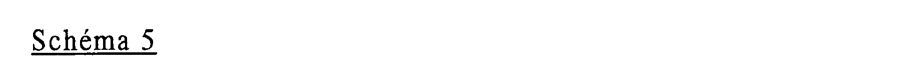

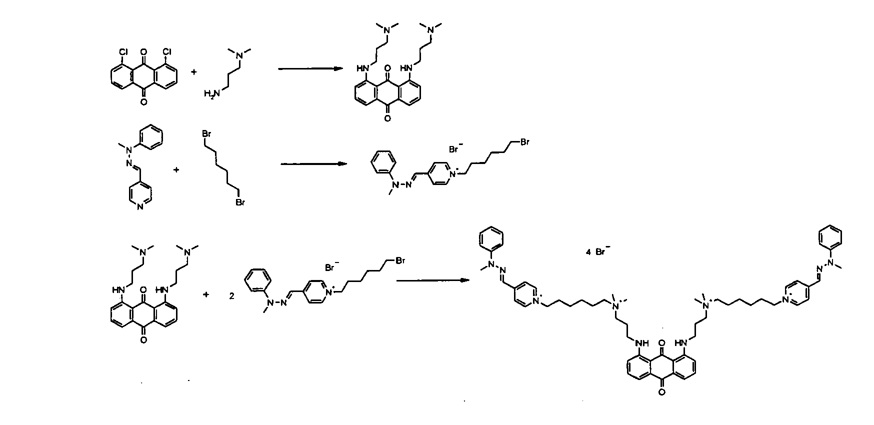

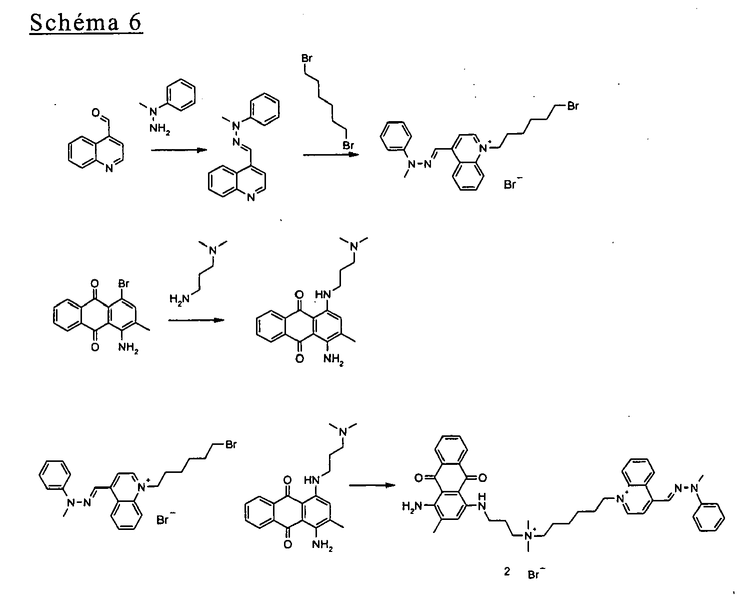

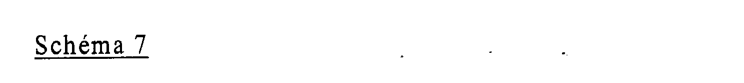

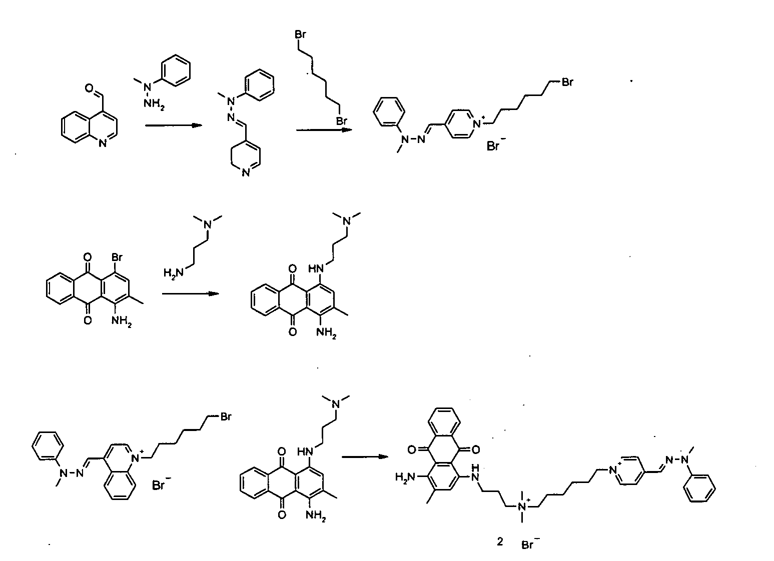

L'invention a également pour objet une composition pour la teinture d'oxydation des fibres kératiniques, et en particulier des fibres kératiniques humaines telles que les cheveux, caractérisée par le fait qu'elle contient, dans un milieu approprié pour la teinture, au moins un colorant mixte tel que défini ci-dessus.

La composition tinctoriale selon l'invention contient notamment de 0,001 à 20% en poids, de préférence de 0,005 à 10% en poids, et encore plus préférentiellement de 0,01 à 5% en poids, d'au moins un colorant mixte de formule (Ia) ou (Ib) selon l'invention ou ses sels ou solvates.

La composition tinctoriale selon l'invention peut contenir un ou plusieurs colorants directs additionnels différents du colorant mixte décrit auparavant.

On peut utiliser les colorants directs classiquement mis en oeuvre dans le domaine de la coloration des fibres kératiniques, et notamment des fibres kératiniques humaines.

A ce titre, on peut notamment citer les colorants nitrés de la série benzénique, les colorants additionnels directs azoïques, les colorants directs méthiniques. Ces colorants directs peuvent être de nature non ionique, anionique ou cationique. De préférence ces colorants directs additionnels sont de nature cationique.

Selon un mode de réalisation particulier de l'invention, la composition tinctoriale comprend une teneur en chacun des colorants directs variant de 0,001 à 10 % en poids par rapport au poids total de la composition tinctoriale.

La composition tinctoriale de l'invention peut aussi contenir une ou plusieurs bases d'oxydation et/ou un ou plusieurs coupleurs, conventionnellement utilisés pour la teinture de fibres kératiniques, et notamment des fibres kératiniques humaines.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition.

La ou les bases d'oxydation présentes dans la composition de l'invention sont en général utilisées chacune en quantité variant de 0,001 à 10 % en poids par rapport au poids total de la composition tinctoriale, de préférence variant de 0,005 à 6 % en poids.

Parmi les coupleurs utilisables, on peut notamment citer les méta-phénylènediamines, les méta-aminophénols, les méta-diphénols, les coupleurs naphtaléniques, les coupleurs hétérocycliques et leurs sels d'addition.

Dans la composition de la présente invention, le ou les coupleurs sont chacun généralement présents en une quantité variant de 0,001 à 10 % en poids par rapport au poids total de la composition tinctoriale, de préférence variant de 0,005 à 6 % en poids.

D'une manière générale, les sels d'addition, notamment des bases d'oxydation et des coupleurs utilisables dans le cadre de l'invention, sont par exemple choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

Le milieu approprié pour la teinture, appelé aussi support de teinture, est un milieu cosmétique généralement constitué par de l'eau ou par un mélange d'eau et d'au moins un solvant organique pour solubiliser les composés qui ne seraient pas suffisamment solubles dans l'eau.

A titre de solvant organique, on peut par exemple citer les monoalcools, linéaires ou ramifiés, de préférence saturés, comprenant 2 à 10 atomes de carbone, tels que l'alcool éthylique, l'alcool isopropylique ; les alcools aromatiques tels que l'alcool benzylique, l'alcool phényléthylique ; les polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol, l'hexylèneglycol (2-méthyl 2,4-pentanediol), le néopentylglycol et le 3-méthyl-1,5-pentanediol ; ainsi que les alkyléthers de diéthylèneglycol, notamment en C₁-C₄, comme par exemple, le monoéthyléther ou le monobutyléther du diéthylèneglycol, seuls ou en mélange.

Les solvants sont, de préférence, présents dans des proportions allant de 1 à 40 % en poids environ par rapport au poids total de la composition tinctoriale, et encore plus préférentiellement de 5 à 30 % en poids environ.

La composition tinctoriale conforme à l'invention peut également renfermer divers adjuvants utilisés classiquement dans les compositions pour la teinture des fibres kératiniques, notamment humaines et en particulier les cheveux, tels que des agents tensioactifs anioniques, cationiques, non-ioniques, amphotères, zwitterioniques ou leurs mélanges ; des polymères anioniques, cationiques, non ioniques, amphotères, zwitterioniques ou leurs mélanges ; des agents épaississants minéraux ou organiques, et en particulier les épaississants associatifs polymériques anioniques, cationiques, non ioniques et amphotères ; des agents antioxydants ; des agents de pénétration ; des agents séquestrants ; des parfums ; des tampons ; des agents dispersants ; des agents de conditionnement tels que par exemple des silicones volatiles ou non volatiles, modifiées ou non modifiées ; des agents filmogènes ; des céramides ou pseudo-céramides ; des agents conservateurs ; des agents opacifiants, etc.

Les adjuvants ci dessus sont en général présents en quantité variant, pour chacun d'eux, de 0,01 à 20 % en poids par rapport au poids de la composition.

La composition de l'invention peut en outre contenir au moins un agent oxydant.

Les agents oxydants classiquement utilisés pour la teinture d'oxydation des fibres kératiniques, notamment humaines, sont par exemple le peroxyde d'hydrogène, le peroxyde d'urée, les bromates ou ferricyanures de métaux alcalins, les persels tels que les perborates et les persulfates de métaux alcalins ou alcalino-terreux, comme le sodium, le potassium, le magnésium, seuls ou en mélanges, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition de l'invention peut de plus comprendre au moins un agent alcalin qui peut être choisi parmi ceux utilisés classiquement dans le domaine cosmétique.

Parmi ces agents alcalins, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (A) suivante : 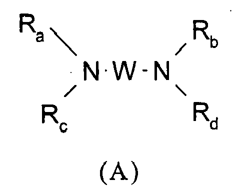 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

Le pH de la composition tinctoriale de l'invention est de préférence compris entre 8 et 11.

Bien entendu, l'homme de l'art veillera à choisir ce ou ces éventuels composés complémentaires de manière telle que les propriétés avantageuses attachées intrinsèquement à la composition selon l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, notamment humaines, et plus particulièrement des cheveux.

Le procédé de la présente invention est un procédé dans lequel on applique sur les fibres, sèches ou non, la composition selon la présente invention telle que définie précédemment.

Plus particulièrement, le procédé de coloration met en oeuvre les étapes suivantes :
- on applique ladite composition comprenant le colorant mixte, sur de fibres sèches ou non, éventuellement en présence d'au moins un agent oxydant,
- on laisse poser pendant une durée suffisante pour obtenir la coloration souhaitée,
- on rince éventuellement les fibres,
- on lave les fibres et on les rince,
- on sèche ou on laisse sécher les fibres.

Selon une première variante, la composition appliquée sur les fibres kératiniques, ne comprend pas d'agent oxydant. Cette variante est particulièrement appropriée, lorsque la composition tinctoriale comprend au moins un colorant mixte selon l'invention et éventuellement au moins un colorant direct additionnel.

Selon une deuxième variante de l'invention, le procédé est mis en oeuvre avec au moins un agent oxydant. Cette deuxième variante est appropriée quelle que soit la nature des colorants présents (colorant mixte, colorant direct additionnel, bases d'oxydation et/ou coupleurs). Un tel procédé permet d'obtenir un éclaircissement de la fibre traitée.

Selon cette deuxième variante, l'agent oxydant peut être ajouté à la composition tinctoriale au moment de l'emploi ou bien il peut encore être mis en oeuvre à partir d'une composition oxydante le comprenant, appliquée simultanément ou séquentiellement à la composition tinctoriale comprenant le colorant mixte. Dans ce dernier cas, l'agent oxydant est présent dans une composition différente de celle comprenant le colorant mixte.

Selon un mode de réalisation particulier, la composition comprenant le colorant mixte est mélangée, de préférence au moment de l'emploi, à une composition contenant, dans un milieu approprié pour la teinture, au moins un agent oxydant, cet agent oxydant étant présent en une quantité suffisante pour obtenir l'éclaircissement souhaité.

Le mélange obtenu est ensuite appliqué sur les fibres kératiniques.

Après un temps de pose suffisant pour obtenir la coloration désirée, habituellement variant de 3 à 50 minutes environ, de préférence 5 à 30 minutes environ, les fibres kératiniques sont de préférence rincées, puis lavées au shampooing, rincées à nouveau puis séchées ou laissées sécher.

Par ailleurs, de manière classique la composition est appliquée et laissée agir à une température allant de 15 à 80°C, de préférence de 15 à 40°C.

La composition oxydante peut également renfermer divers adjuvants utilisés classiquement dans les compositions pour la teinture des fibres kératiniques, notamment humaines, et tels que définis précédemment.

Le pH de la composition oxydante renfermant l'agent oxydant est tel qu'après mélange avec la composition tinctoriale, le pH de la composition résultante appliquée sur les fibres kératiniques (soit en d'autres termes la composition prête à l'emploi) varie de préférence de 7 à 12 environ, et encore plus préférentiellement de 8 à 11. Il peut être ajusté à la valeur désirée au moyen d'agent(s) acidifiant(s) ou alcalinisant(s).

Parmi les agents acidifiants, on peut citer, à titre d'exemples, les acides minéraux ou organiques comme l'acide chlorhydrique, l'acide orthophosphorique, l'acide sulfurique, l'acide acétique.

Pour ce qui concerne les composés alcalinisants, on pourra se reporter à la liste précédemment indiquée.

La composition prête à l'emploi, soit en d'autres termes la composition qui est finalement appliquée sur les fibres kératiniques, peut se présenter sous des formes diverses, telles que sous forme de liquides, de crèmes, de gels ou sous toute autre forme appropriée pour réaliser une teinture des fibres kératiniques, notamment des fibres kératiniques humaines, telles que les cheveux.

L'invention a aussi pour objet un dispositif à plusieurs compartiments dans lequel au moins un premier compartiment comprend une composition tinctoriale comprenant au moins un colorant mixte tel que décrit auparavant, et éventuellement au moins un colorant direct différent du colorant mixte, éventuellement au moins une base d'oxydation, éventuellement au moins un coupleur, et un autre compartiment comprend un agent oxydant.

Il est à noter que le ou les colorants mixtes, éventuellement le colorant direct additionnel, la ou les bases d'oxydation, le ou les coupleurs, peuvent se trouver dans un même compartiment ou dans plusieurs ; un même compartiment pouvant comprendre un seul type de colorant (mixte, direct additionnel, oxydation) ou une combinaison de plusieurs d'entre eux.

Ce dispositif peut être équipé d'un moyen permettant de délivrer sur les fibres à traiter, le mélange souhaité, tel que les dispositifs décrits dans le brevet FR 2 586 913.

L'invention a également pour objet l'utilisation d'au moins colorant mixte tel que défini ci-dessus pour la coloration des fibres kératiniques, telles que les cheveux.

L'invention concerne enfin l'utilisation de la composition tinctoriale telle que définie ci-dessus pour la coloration des fibres kératiniques, telles que les cheveux.

Les exemples qui suivent sont destinés à illustrer l'invention sans toutefois en limiter la portée.

### EXEMPLES

### Exemple 1 : anthraquinone reliée à deux chromophores azoïques en série azo-imidazolium - colorant 1

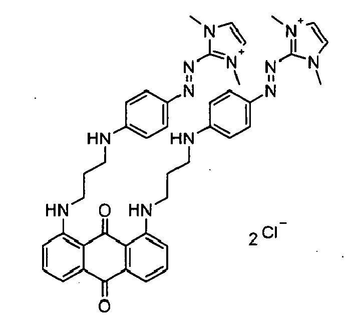

### Voie de synthèse

Première étape : préparation de la 1,8-bis[(3-aminopropyl)amino]anthra-9,10-quinone 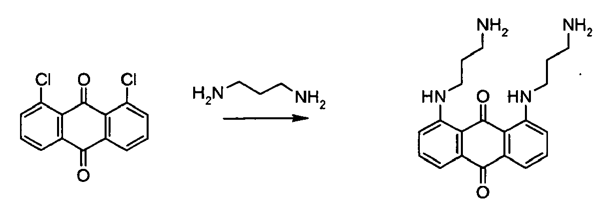 Un mélange de 1,8-dichloroanthra-9,10-quinone et un excès de 1,3-propanediamine est agité et chauffé à 90°C pendant 24 heures. Il est refroidi puis versé dans l'ether isopropylique. La solution obtenue est lavée avec une solution de soude diluée, séchée sur sulfate de sodium et concentré sous vide. Le produit violet obtenu sous forme de pâte est purifié par chromatographie sur gel de silice (acetate d'éthyle, éthanol et triéthylamine). On recueille une poudre violet. Les analyses sont conformes à la structure de la 1,8-bis[(3-aminopropyl)amino]anthra-9,10-quinone.
Deuxième étape : double substitution d'un groupement MeO sur le chlorure de 2-[(4-méthoxyphényl)diazényl]-1,3-diméthyl-1H-imidazol-3-ium par les groupement amino de la 1,8-bis[(3-aminopropyl)amino] anthra-9,10-quinone : 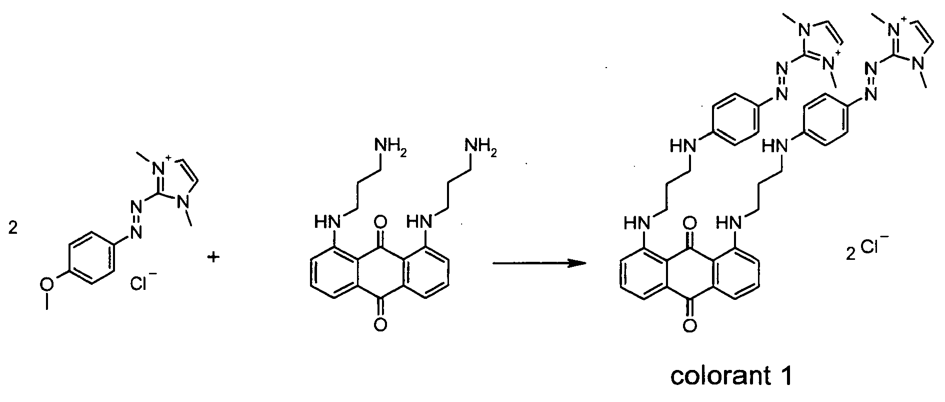 1.89 g de chlorure de 2-[(4-méthoxyphényl)diazényl]-1,3-diméthyl-1H-imidazol-3-ium et la 1,8-bis[(3-aminopropyl)amino]anthra-9,10-quinone (rapport stoechiométrique de deux pour un) sont mélangés en solution dans un mélange de pentanol (7 mL) et de dichlorométhane (70 mL), le mélange ainsi obtenu est chauffé à 80°C (le dichlorométhane distille progressivement) pendant 24 heures, puis précipité après refroidissement par addition supplémentaire de dichlorométhane (800 mL). On recueille après essorage, rinçage au dichlorométhane et séchage, 1.3 g de poudre brun rouge. Les analyses sont conformes à la structure du produit attendu (colorant 1).
Exemple 2 : colorant anthraquinonique relié à deux chromophores hydrazones en série 4-pyridinium - variante pour laquelle le bras de liaison est cationique - colorant 2

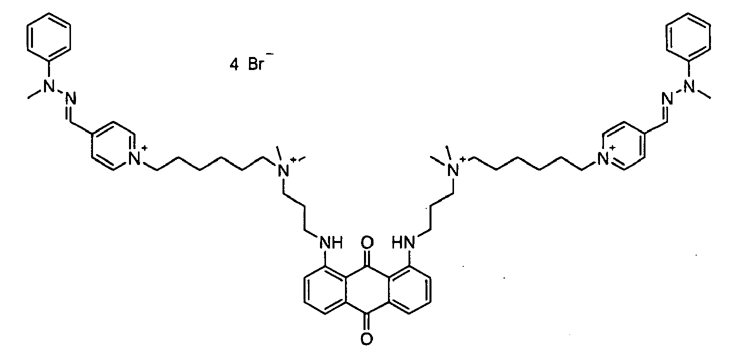

### Première étape : préparation de la 1,8-bis[(3-diméthylaminopropyl) amino]anthra-9,10-quinone

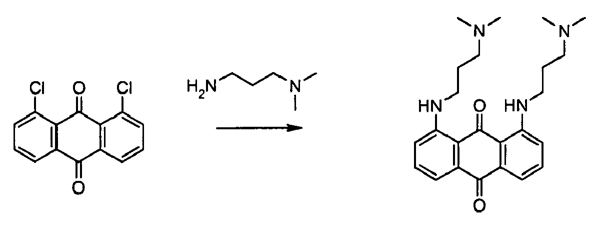

Un mélange de 25 g de 1,8-dichloroanthra-9,10-quinone et de 150 g de N,N-diméthylamino-1,3-propylènediamine est agité et chauffé à 90°C pendant 24 heures. I1 est refroidi puis versé dans 1 L d'éther isopropylique. La solution obtenue est lavée avec une solution de soude diluée, séchée sur sulfate de sodium et concentré sous vide. Le produit violet obtenu sous forme de pâte (31 g) est purifié par chromatographie sur gel de silice (acétate d'éthyle, éthanol et triéthylamine) puis cristallisation dans l'éther de pétrole. On recueille 23,5 g de cristaux violets. Les analyses sont conformes à la structure de la 1,8-bis[(3-diméthylaminopropyl) amino]anthra-9,10-quinone.

### Deuxième étape : formation du bromure de 1(6-bromohexyl)-4-{(E)-[méthyl(phényl)hydrazono]méthyl}pyridinium

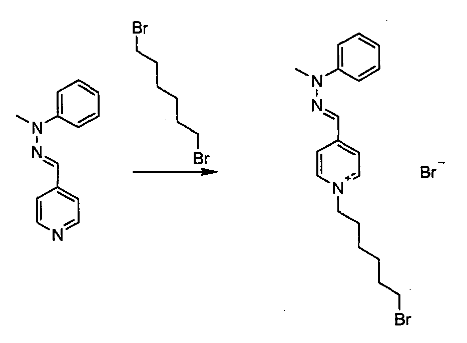

On solubilise la méthyl(phényl)hydrazone de l'isonicotinaldéhyde (80,1 g) dans 250 mL de toluène. Le mélange est chauffé à 80°C puis on additionne en 10 minutes une solution de 1,6-dibromohexane (473 g) dans 750 mL de toluène. Après 4,5 heures le milieu réactionnel est refroidi puis filtré. Le précipité obtenu est lavé avec 200 mL de toluène puis 100 mL d'éther de pétrole. Il est solubilisé dans 1 L de dichlorométhane puis extrait à l'eau. La phase organique est lavée 4 fois à l'eau, séchée sur sulfate de magnésium, filtrée puis concentrée. On obtient une huile marron qui est reprise par 50 mL de toluène. On obtient un précipité jaune qui est séparé par filtration puis séché pour conduire à 95,1 g du produit attendu sous la forme d'une poudre jaune. Les analyses l'indiquent conforme à l'attendu (bromure de 1(6-bromohexyl)-4-{(E)-[méthyl(phényl)hydrazono]méthyl}pyridinium)

### Troisième étape : réaction entre les deux composés préparés suivant les deux étapes précédentes

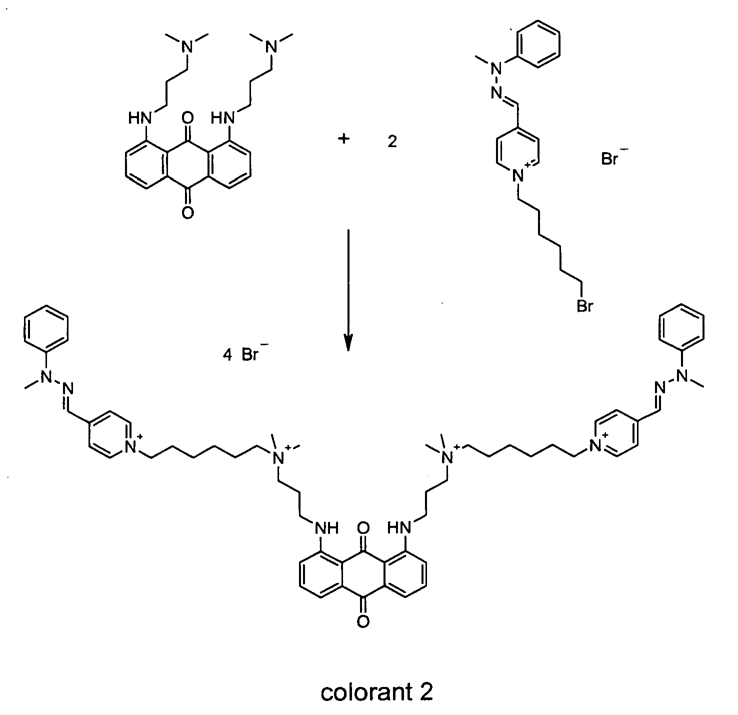

Le bromure de 1(6-bromohexyl)-4-{(E)-[méthyl(phényl)hydrazono] méthyl}pyridinium (28.78 g) et le 1,8-bis[(3-diméthylaminopropyl) amino]anthra-9,10-quinone (11.73 g) sont solubilisés dans 250 mL de N,N-diméthylformamide. Le mélange réactionnel ainsi formé est agité et chauffé à 70°C pendant 24 h. Il est refroidi puis versé dans 3 L d'acétone et le produit attendu précipite. Après filtration, on recueille 35.2 g d'une poudre noire contenant plus de 85% de produit attendu. Une purification plus poussée est réalisée par chromatographie liquide-liquide (n-butanol/eau). On recueille ainsi 17.5 g de produit pur (poudre noir brillant). Les analyses l'indiquent conforme à l'attendu (colorant 2).
Cette purification permet également d'obtenir un produit formé minoritairement et correspondant au produit de monosubstitution (voir colorant 3)

### Exemple 3 : colorant anthraquinonique relié à un chromophore hydrazone en série 4-pyridinium - variante pour laquelle le bras de liaison est cationique - colorant 3

La purification menée lors de la préparation du colorant 2 a permis également d'obtenir le produit suivant sous forme de poudre noire (0.5 g). Les analyses l'indiquent conforme à la structure du colorant 3:

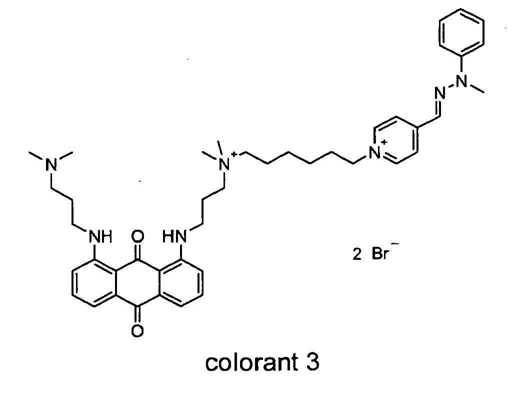

### Exemple 4: colorant anthraquinonique relié à un chromophore hydrazone - variante pour laquelle le bras de liaison est cationique-colorant 4

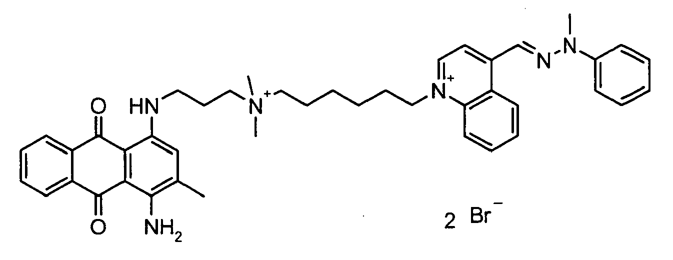

Voie de synthèse
Première étape : préparation de la 1-amino-4-{[3-(diméthylamino) propyl]amino}-2-méthylanthra-9,10-quinone 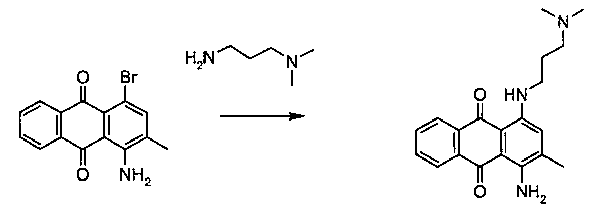 Conditions de synthèse
Dans un ballon de 250 mL, sont introduits successivement la 1-amino-4-bromo-3-méthylanthra-9,10-quinone 6.32 g, 50 ml de NMP (N-méthylpyrrolidone) puis la N,N-diméthylamino-1,3-propylènediamine 25 mL. Le mélange est agité et porté à 80°C pendant 18 h.
Après un contrôle ccm (AcOEt pur : RF 0 ; eau/EtOH/AcOH 2 :2 :1 : RF 0.6) qui indique une réaction complète, le mélange réactionnel est versé sur 1 L d'eau.
Le précipité formé est solubilisé dans une solution aqueuse acide chlorhydrique (0,25M, 150 mL), laquelle est lavée par du dichlorométhane (150 mL), filtrée, puis neutralisée à la soude (10N) pour faire reprécipiter le produit attendu. Le précipité ainsi obtenu est filtré et lavé à l'eau, repris au dichlorométhane (1.5 L), séché sur sulfate de sodium et concentré sous vide.
On obtient 3,24 g de poudre bleu-violet ; Les analyses indiquent que le produit est conforme (1-amino-4-{[3-(diméthylamino) propyl]amino}-2-méthylanthra-9,10-quinone).

### Deuxième étape : préparation de la quinoline-4-carbaldehyde methyl(phenyl)hydrazone

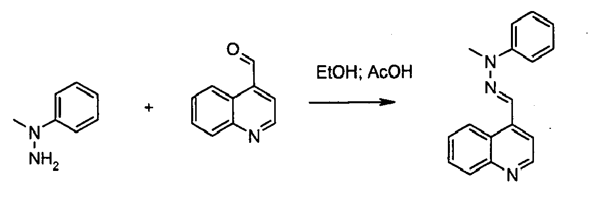

Conditions de synthèse
Dans un tricol de 250 mL muni d'un thermomètre et surmonté d'un réfrigérant, sont introduits 4.12 g de N-méthylphénylhydrazine, 27 mL d'éthanol puis 0.5 mL d'acide acétique. Le milieu réactionnel est agité et refroidi à 10°C. 5.30 g de quinoline 4-carboxaldéhyde sont ensuite ajoutés, en 5 minutes. Le milieu réactionnel prend en masse, il est ensuite agité à 70°C pendant 24 h.
Après un contrôle ccm (AcOEt / heptane 4 :6 RF 0.4) qui indique que la réaction est achevée, le mélange réactionnel est versé chaud sur 1 L de mélange eau/glace. Un précipité orangé se forme. Il est essoré, lavé à l'eau puis à l'éther de pétrole et séché sous vide. On obtient 8.03 g de poudre orange. Les analyses indiquent que le produit est conforme à l'attendu (quinoline-4-carbaldehyde methyl(phenyl)hydrazone).

### Troisième étape : préparation du bromure de 1-(6-bromohexyl)-4-{(E)-[méthyl(phényl)hydrazono]méthyl}quinolinium

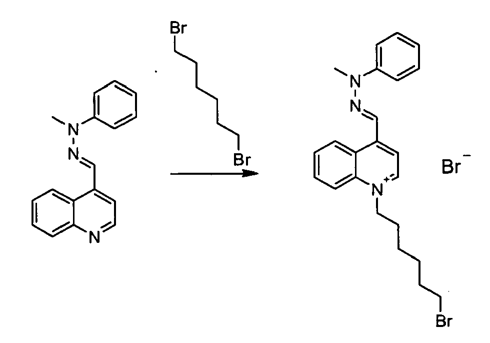

Conditions de synthèse
Dans un tricol de 250 mL muni d'un réfrigérant, 90 mL de dibromohexane sont agités et chauffés à 30°C puis 7.5 g de quinoline-4-carbaldéhyde méthyl(phényl)hydrazone sont progressivement ajoutés. Le mélange, homogène après quelques minutes est porté à 80°C pour 5 h.
Après une ccm de contrôle (CH₂Cl₂/ MeOH 8 :2 RF 0.6) qui indique que la réaction est achevée, le produit est essoré à chaud, lavé deux fois au toluène, essoré et lavé à l'éther de pétrole avant séchage. 9.2 g de produit sont ainsi obtenus sous forme de poudre orange, indiqué conforme à l'attendu par les analyses (bromure de 1-(6-bromohexyl)-4-{(E)-[méthyl(phényl)hydrazono]méthyl}quinolinium).

### Quatrième étape :

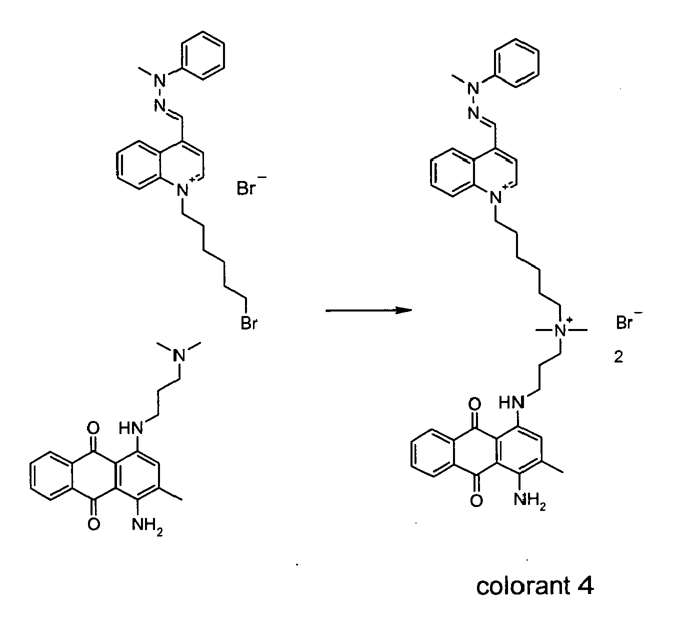

Conditions de synthèse
Le bromure de 1-(6-bromohexyl)-4-{(E)-[méthyl(phényl)hydrazono] méthyl}quinolinium (1.5 g) est solubilisé dans 25 mL de DMF (diméthylformamide), la 1-amino-4-{[3-(diméthylamino)propyl]amino}-2-méthylanthra-9,10-quinone (1,0 g) est ajoutée et le mélange est agité et chauffé pendant 48h à 80°C, dans un ballon muni d'un réfrigérant et d'une garde à chlorure de calcium. Après refroidissement et précipitation du mélange réactionnel dans l'acétone, le produit est purifié par chromatographie liquide-liquide (n-butanol/eau). On recueille 2,2 g de produit sous forme de poudre noire. Les analyses l'indiquent conforme à l'attendu (colorant 4).

### Exemple 5 : colorant anthraquinonique relié à un chromophore hydrazone - variante pour laquelle le bras de liaison est cationique - colorant 5

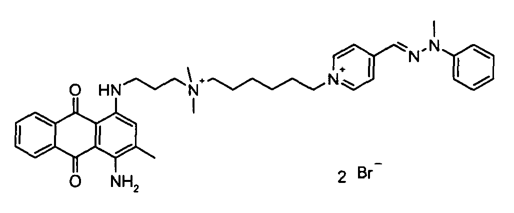

Voie de synthèse : 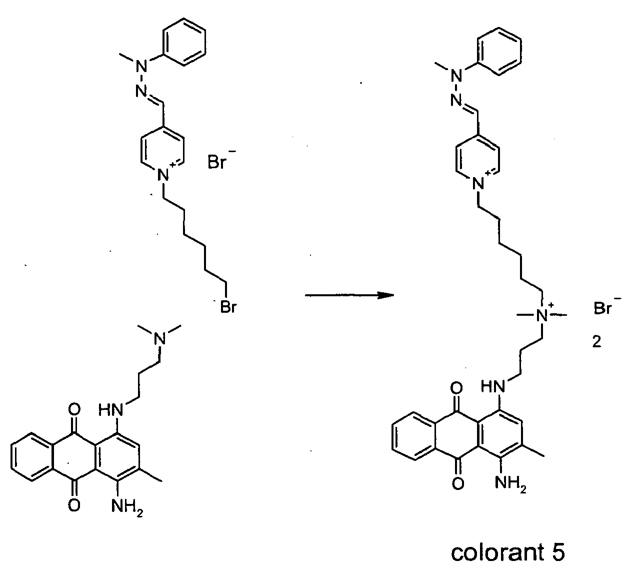 Conditions de synthèse
Le bromure de 1-(6-bromohexyl)-4-{(E)-[méthyl(phényl)hydrazono] méthyl}pyridinium (1,37 g) est solubilisé dans 25 mL de DMF (diméthylformamide), la 1-amino-4-{[3-(diméthylamino)propyl]amino}-2-méthylanthra-9,10-quinone (1,00 g) est ajoutée et le mélange est agité et chauffé pendant 18 h à 80°C, dans un ballon muni d'un réfrigérant et d'une garde à chlorure de calcium. Le solvant est éliminé par distillation sous vide, la pâte obtenue est malaxée avec du dichlorométhane. Le solide ainsi formé est essoré et lavé plusieurs fois au dichlorométhane. On recueille 1,96 g de poudre vert foncé. Les analyses l'indiquent conforme à l'attendu (colorant 5).

### Exemple 6 : colorant anthraquinonique relié à un chromophore azoïque - colorant 6

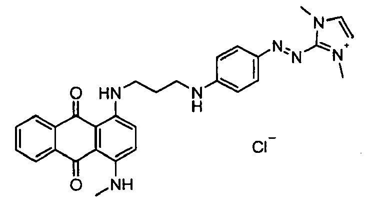

Voie de synthèse : 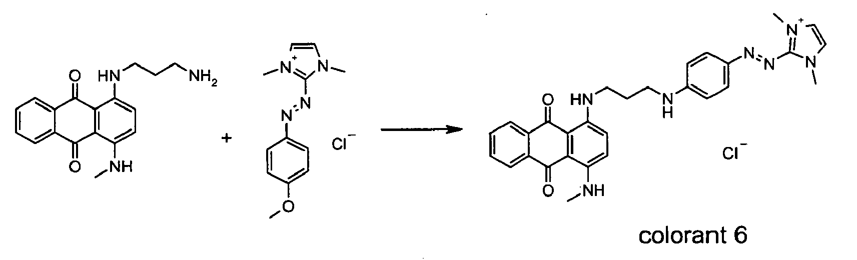 Conditions de synthèse
Le chlorhydrate de-[(3-aminopropyl)amino]-4-(méthylamino)anthra-9,10-quinone (2,00 g) et le chlorure de 2-[(4-méthoxyphényl)diazényl]-1,3-diméthyl-1H-imidazol-3-ium (1.78 g) sont mélangés à 50 mL de méthanol et 50 mL d'isopropanol. Diisopropyléthylamine (1,50 g) est ensuite ajouté et le mélange est agité et chauffé à reflux pendant 24 h. Après évaporation du méthanol et ajout d'isopropanol, on recueille un précipité rouge brique (2,2 g après filtration et séchage). Les analyses indiquent le produit conforme à l'attendu (colorant 6).

### Exemples de teinture

Les compositions tinctoriales suivantes sont préparées :

| | |
|---|---|
| Colorant | 10-3 mole |
| Support de teinture | (*) |
| Eau déminéralisée q.s.p. | 100g |

| | |
|---|---|
| (*) : support de teinture (1) pH 7 ou (2) pH 9,5 | |

### Support de teinture (1) pH7 :

| | |
|---|---|
| Alcool éthylique à 96° | 20,8 g |
| Sel pentasodique de l'acide diéthylène-triamine-pentaacétique en solution aqueuse à 40% | 0,48 g M.A |
| Alkyl en C8-C10 polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

### Support de teinture (2) pH9.5 :

| | |
|---|---|
| Alcool éthylique à 96° | 20,8 g |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Pour les colorations dans les conditions non éclaircissantes (sans oxydant), ces compositions sont appliquées directement sur les cheveux.

Pour les colorations dans les conditions éclaircissantes on utilise un milieu oxydant. Dans ce cas, au moment de l'emploi, chaque composition est mélangée avec un poids égal d'eau oxygénée à 20 volumes (6% en poids). On obtient un pH final de 7 ou 9.5.

Chaque mélange obtenu est appliqué sur des mèches de cheveux gris à 90 % de blancs avec un rapport de bain 6 :1. Après 30 minutes de pose, les mèches sont rincées, lavées avec un shampooing standard, rincées à nouveau puis séchées.

### Les résultats de teinture suivants ont été obtenus

| | pH7 (sans oxydant) | pH 9,5 (sans oxydant) | pH7 (avec oxydant) | pH 9,5 (avec oxydant) |
|---|---|---|---|---|
| Colorant 1 | brun-rouge | brun-rouge | brun-rouge | brun-rouge |
| Colorant 2 | brun doré | brun doré | brun doré | brun doré |
| Colorant 4 | brun | brun | brun | brun |
| Colorant 5 | vert-brun | vert-brun | vert-brun | vert-brun |

Les mèches ainsi colorées sont soumises à un test de résistance aux lavages, qui consiste à effectuer 12 shampooings (avec un shampooing standard) et à évaluer la couleur après ces 12 shampooings. Apres 12 shampooings les mèches sont toujours colorées.

## Revendications

1. Colorant direct mixte cationique comprenant un chromophore anthraquinone et au moins un chromophore azoïque cationique et/ou au moins un chromophore hydrazone cationique, le chromophore anthraquinone étant lié à et/ou aux autres chromophores cationiques par l'intermédiaire d'au moins un bras de liaison.

2. Colorant selon la revendication 1, **caractérisé en ce que** les chromophores absorbent dans le domaine visible entre 400 et 800 nm.

3. Colorant selon la revendication 1 ou 2, **caractérisé en ce que** le colorant mixte comprend deux à trois chromophores.

4. Colorant selon l'une quelconque des revendications 1 à 3, **caractérisé en ce que** le ou les chromophores cationiques sont des chromophores comprenant au moins un atome d'azote quaternisé.

5. Colorant selon l'une quelconque des revendications 1 à 4, **caractérisé en ce qu'**il répond aux formules (Ia) ou (Ib) suivantes : 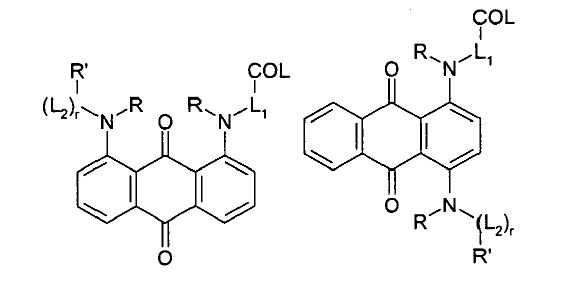 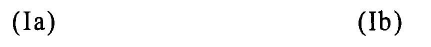 dans lesquelles :
- L₁ représente un bras de liaison, cationique ou non, reliant l'atome d'azote de l'anthraquinone au groupement COL par l'intermédiaire d'un atome de carbone, d'oxygène ou d'azote quaternisé ou non ;
- L₂ représente un bras de liaison, cationique ou non, reliant le deuxième azote de l'anthraquinone au groupement R' par l'intermédiaire d'un atome de carbone, d'oxygène ou d'azote quaternisé ou non ;
- r est égal à 0 ou 1 ;
- les groupements R représentent, indépendamment l'un de l'autre, un atome d'hydrogène ou un radical alkyle en C₁-C₄ éventuellement substitué ; le cas échéant, l'un au moins des groupements R peut être engagés dans un cycle saturé ou insaturé, éventuellement aromatique, comprenant de 5 à 7 chaînons, avec L₁ ou L₂ ;
- R' représente :
- un atome d'hydrogène
- une chaîne hydrocarbonée en C₁-C₁₂ linéaire ou ramifiée, éventuellement substituée, éventuellement interrompue ou terminée par un ou plusieurs groupements : amino, mono ou di-alkylamino éventuellement substitué, alkyl ou arylammonium, éventuellement substitué, hétérocycle à 5 ou 6 chaînons, saturé ou insaturé, comprenant au moins un atome d'azote quaternisé engagé dans ledit hétérocycle, ou
- un groupement COL ;
- COL représente un radical colorant appartenant à la famille des azoïques cationiques ou des hydrazones cationiques,
lorsque COL est un radical azoïque cationique, il répond à la formule générale suivante : 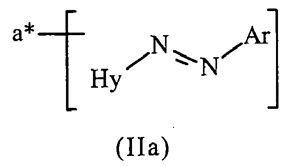 lorsque COL est un radical hydrazone cationique, il répond à la formule générale suivante : 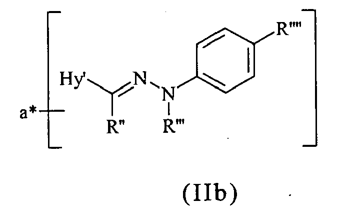
- a* représente la liaison reliant COL à L₁ ;
- Hy représente un hétérocycle cationique de formule générale : 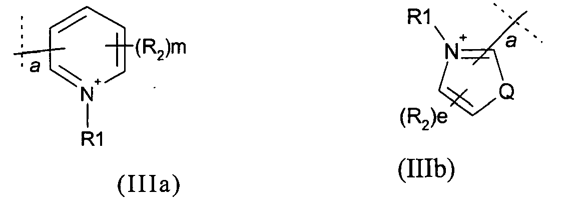
- Hy' représente un hétérocycle cationique de formule générale IIIa
- R₁ indépendamment les uns des autres représentent une chaîne hydrocarbonée en C₁-C₁₆, linéaire ou ramifiée, saturée ou insaturée, pouvant former un ou plusieurs cycles carbonés comportant de 3 à 7 chaînons, éventuellement condensés avec le cycle aromatique, éventuellement substituée, éventuellement interrompue par un ou plusieurs groupements choisis parmi les hétéroatomes tels que l'oxygène, l'azote ou le soufre, et le groupement carbonyle; R₁ ne comportant pas de fonction nitro, nitroso, peroxyde et diazo, R₁ étant directement rattaché à l'atome d'azote quaternisé ou non, du cycle hétéroaromatique par l'intermédiaire d'un atome de carbone.
- R₂ indépendamment les uns des autres, représentent :
- une chaîne hydrocarbonée en C₁-C₁₆, linéaire ou ramifiée, saturée ou insaturée, aromatique ou non, pouvant former un ou plusieurs cycles carbonés comportant de 3 à 6 chaînons, éventuellement substituée, éventuellement interrompue par un ou plusieurs hétéroatomes
ou par un ou plusieurs groupements portant au moins un hétéroatome, de préférence choisis parmi l'oxygène, l'azote, le soufre, tels que -CO-,
- SO₂- ou leurs combinaisons ;
• un groupement hydroxyle,
• un groupement alkyl-oxy en C₁-C₄ ; un groupement (poly)hydroxyalcoxy en C₂-C₄,
• un groupement alcoxycarbonyle R₁₁O-CO-, dans lequel R₁₁ représente un radical alkyle en C₁-C₄, un radical alkylcarbonyloxy R₁₂CO-O-, dans lequel R₁₂ représente un radical alkyle en C₁-C₄ ;
• un groupement amino ; un groupement amino substitué par un ou deux radicaux alkyles en C₁-C₄, identiques ou différents, éventuellement porteurs d'au moins un groupement hydroxyle, les deux radicaux alkyles formant éventuellement avec l'atome d'azote auquel ils sont rattachés, un hétérocycle à 5 ou 6 chaînons éventuellement porteur d'un autre hétéroatome identique ou différent de l'azote, par exemple l'oxygène, le soufre ;
• un groupement alkylcarbonylamino R₁₃CO-NR₁₄-, dans lequel les radicaux R₁₃ et R₁₄, indépendamment l'un de l'autre, représentent un radical alkyle en C₁-C₄; un groupement carbamoyle (R₁₅)₂N-CO, dans lequel les radicaux R₁₅, indépendamment l'un de l'autre, identiques ou non, représentent un atome d'hydrogène, un radical alkyle en C₁-C₄; un groupement uréido (R₁₆)₂N-CO-NR₁₇-, dans lequel les radicaux R₁₆ et R₁₇, indépendamment les uns des autres, représentent un atome d'hydrogène, un radical alkyle en C₁-C₄ ;
• un groupement sulfonamide (R₁₈)₂N-SO₂-, dans lequel les radicaux R₁₈, indépendamment l'un de l'autre, représentent un atome d'hydrogène, un radical alkyle en C₁-C₄; un groupement alkylsulfonylamino R₁₉SO₂-NR₂₀-, dans lequel les radicaux R₁₉ et R₂₀, indépendamment l'un de l'autre, représentent un atome d'hydrogène, un radical alkyle en C₁-C₄ ;
• un groupement guanidinium (R₂₁)₂N-C(=NH₂⁺)-NR₂₂-, dans lequel les radicaux R₂₁ et R₂₂, indépendamment l'un de l'autre, représentent un atome d'hydrogène, un radical alkyle en C₁-C₄;
• un groupement alkylsulfonyle R₂₃-SO₂-, dans lequel R₂₃ représente un radical alkyle en C₁-C₄ ;
• un groupement nitro ; un groupement cyano ; un atome d'halogène, de préférence le chlore, le fluor ;
• un groupement phényle éventuellement substitué ;
deux radicaux R₂, portés par des atomes de carbone adjacents peuvent former ensemble avec l'atome de carbone auquel chacun est rattaché, un cycle condensé aromatique éventuellement substitué;
- m représente un entier compris entre 0 et 4 ; lorsque m est inférieur à 4, alors le ou les atomes de carbone de l'hétérocycle non substitués portent un atome d'hydrogène ;
- e est un entier compris entre 0 et 2 ; lorsque e est inférieur à 2, alors le ou les atomes de carbone de l'hétérocycle non substitués portent un atome d'hydrogène ;
- Q représente NR₁, O ou S ;
- la liaison α issue des formules (IIIa) ou (IIIb) relie le groupement Hy au groupement azoïque -N=N-Ar de la formule (IIa) ou le groupement Hy' au groupement hydrazone -CR"=N-NR"' de la formule (IIb); dans le cas des formules (IIIa) ou (IIIb), et lorsque deux radicaux R₂ portés par deux atomes de carbone adjacents forment un cycle aromatique, la liaison a peut relier le groupement Hy au groupement azoïque -N=N-Ar de la formule (IIa) ou le groupement Hy' au groupement hydrazone -CR"=N-NR"' de la formule (IIb) par l'intermédiaire dudit cycle aromatique ;
- Ar représente un cycle aromatique du type : 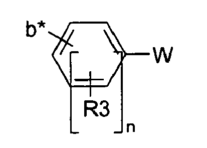
dans lequel:
- b* représente la liaison reliant Ar à NR"' de la formule (IIb) ou à la fonction azoïque de la formule (IIa) ;
- n est un nombre entier valant de 0 à 4 ; lorsque n est inférieur à 4, alors le ou les atomes de carbone du cycle aromatique non substitués portent un atome d'hydrogène ;
R₃ identiques ou différents, représentent :
. un radical alkyle en C₁-C₁₆, éventuellement substitué, éventuellement interrompu par un ou plusieurs hétéroatomes ou par un
ou plusieurs groupements comprenant au moins un hétéroatome, de préférence choisi parmi l'oxygène, l'azote, le soufre, tels que -CO-,
- SO₂- ou leurs combinaisons ;
• un groupement hydroxyle,
• un groupement alcoxy en C₁-C₄ ; un groupement (poly)hydroxyalcoxy en C₂-C₄ ;
• un groupement alcoxycarbonyle R₃₁O-CO-, dans lequel R₃₁ représente un radical alkyle en C₁-C₄ ; un radical alkylcarbonyloxy R₃₂CO-O-, dans lequel R₃₂ représente un radical alkyle en C₁-C₄ ;
• un radical alkylcarbonyle R₃₃-CO-, dans lequel R₃₃ représente un radical alkyle en C₁-C₄ ;
• un groupement amino ; un groupement amino substitué par un ou deux radicaux alkyle en C₁-C₄ , identiques ou différents, éventuellement porteurs d'au moins un groupement hydroxyle ; les deux radicaux alkyle formant éventuellement avec l'atome d'azote auquel ils sont rattachés, un hétérocycle renfermant 1 à 3 hétéroatomes, de préférence 1 à 2 hétéroatomes, choisis parmi N, O, S, de préférence N, comprenant de 5 à 7 chaînons, saturé ou insaturé, aromatique ou non, éventuellement substitué ;
• un groupement alkylcarbonylamino R₃₄CO-NR₃₅-, dans lequel le radical R₃₄ représente un radical alkyle en C₁-C₄ et le radical R₃₅ représente un atome d'hydrogène ou un radical alkyle en C₁-C₄ ;
• un groupement aminocarbonyle (R₃₆)₂N-CO-, dans lequel les radicaux R₃₆ indépendamment l'un de l'autre, représentent un atome d'hydrogène, un radical alkyle en C₁-C₄ ;
• un groupement uréido N(R₃₇)₂-CO-NR₃₈-, dans lequel les radicaux R₃₇ et R₃₈, indépendamment les uns des autres, représentent un atome d'hydrogène, un radical alkyle en C₁-C₄ ;
• un groupement aminosulfonyle (R₃₉)₂N-SO₂-, dans lequel les radicaux R₃₉ indépendamment l'un de l'autre, représentent un atome d'hydrogène, un radical alkyle en C₁-C₄ ;
• un groupement alkylsulfonylamino R₄₀SO₂-NR₄₁-, dans lequel les radicaux R₄₀ et R₄₁, indépendamment l'un de l'autre, représentent un atome d'hydrogène, un radical alkyle en C₁-C₄ ;
• un groupement thiol HS- ;
• un groupement alkylthio R₄₂S-, dans lequel le radical R₄₂ représente un radical alkyle en C₁-C₄ ;
• un groupement alkylsulfinyle R₄₃-SO-, dans lequel R₄₃ représente un radical alkyle en C₁-C₄ ;
• un groupement alkylsulfonyle R₄₄-SO₂-, dans lequel R₄₄ représente un radical alkyle en C₁-C₄ ;
• un groupement nitro ;
• un groupement cyano ;
• un atome d'halogène, de préférence le chlore, le fluor ;
lorsque n est supérieur ou égal à 2, deux radicaux R₃ adjacents peuvent former avec les atomes de carbone auxquels ils sont rattachés, un cycle secondaire, aromatique ou non à 6 chaînons, éventuellement substitué ;
W représente :
• un atome d'hydrogène,
• un atome d'halogène choisi parmi le brome, le chlore, le fluor de préférence le chlore et le fluor,
• un groupement -NR₅R₆, -OR₇, -NR₄-Ph-NR₅R₆, -NR₄-Ph-OR₇, -O-Ph-OR₇, -O-Ph-NR₅R₆, -SO₂-NR₅R₆, -SO₂-R₅ ; avec :
■ R₄ et R₇, identiques ou non, représentent un atome d'hydrogène, un radical alkyle en C₁-C₂₀, de préférence en C₁-C₁₆ éventuellement substitué, un radical aralkyle en C₁-C₃₀ éventuellement substitué, un radical aryle éventuellement substitué ;
■ R₅ et R₆ identiques ou non, représentent un atome d'hydrogène, un radical alkyle en C₁-C₂₀, de préférence en C₁-C₁₆ éventuellement substitué, un radical phényle éventuellement substitué, un radical aryle
ou aralkyle en C₁-C₃₀ éventuellement substitué, un radical alkylcarbonyle R₄₅-CO-, dans lequel R₄₅ est un radical alkyle en C₁-C₄ éventuellement substitué;
■ R₅ et R₆ formant éventuellement avec l'atome d'azote auquel ils sont rattachés, un hétérocycle renfermant 1 à 3 hétéroatomes choisis parmi N, O, S, de préférence N, comprenant de 5 à 7 chaînons, saturé ou insaturé, aromatique ou non, éventuellement substitué ;
■ R₅ et R₆, indépendamment l'un de l'autre peuvent former avec l'atome de carbone du cycle aromatique adjacent à celui auquel ―N R₅R₆, est rattaché, un hétérocycle saturé à 5 ou 6 chaînons ;
■ Ph représentant un radical phényle éventuellement substitué ;
R" représente
• un atome d'hydrogène,
• un radical alkyle en C₁-C₁₆, éventuellement substitué, éventuellement interrompu par un ou plusieurs hétéroatomes ou par un
ou plusieurs groupements comprenant au moins un hétéroatome, de préférence choisi parmi l'oxygène, l'azote, le soufre, tels que -CO-,
- SO₂- ou leurs combinaisons ;
• un radical aryle ou arylalkyle en C₆-C₃₀, tel que phényle, benzyle, la partie aryle étant éventuellement substituée de préférence par un ou plusieurs groupements identiques ou différents de préférence choisis parmi par un atome de chlore, un groupement amino, un groupement hydroxyle, un groupement alcoxy en C₁-C₂, un groupement amino mono
ou disubstitué par deux radicaux alkyle identiques ou différents éventuellement porteurs d'au moins un groupement hydroxyle ;
R"' représente
• un atome d'hydrogène,
• un radical alkyle en C₁-C₁₆, éventuellement substitué, éventuellement interrompu par un ou plusieurs hétéroatomes ou par un ou plusieurs groupements comprenant au moins un hétéroatome, de préférence choisi parmi l'oxygène, l'azote, le soufre, tels que -CO-, -SO₂- ou leurs combinaisons ;
• un radical aryle ou arylalkyle en C₆-C₃₀, tel que phényle, benzyle, la partie aryle étant éventuellement substituée de préférence par un ou plusieurs groupements identiques ou différents de préférence choisis parmi par un atome de chlore, un groupement amino, un groupement hydroxyle, un groupement alcoxy en C₁-C₂, un groupement amino mono ou disubstitué par deux radicaux alkyle identiques ou différents éventuellement porteurs d'au moins un groupement hydroxyle ; R"" représente un atome d'hydrogène, un atome d'halogène choisi parmi le brome, le chlore, le fluor de préférence le chlore et le fluor, un groupement -OR₇.
la liaison a* se situant :
- soit sur l'un des atomes d'azote quaternisé ou non des formules (IIIa) ou (IIIb),
- soit sur l'un des atomes de carbone des hétérocycles des formules (IIIa) ou (IIIb),
- soit sur l'un des atomes de carbone du cycle aromatique de Ar,
- soit sur l'atome de carbone portant R" ou R''',
- soit sur l'atome d'azote portant les radicaux R₅, R₆,
- soit sur l'atome d'oxygène portant R₇,
auquel cas le radical R₁, R₂, R₃, R", R"', R₅, R₆ ou R₇ concerné est remplacé par une liaison simple reliant L₁ à COL ;
dans le cas ou R' représente le radical COL, L₂ est identique à L₁ et est relié de la même manière à R' que L₁ à COL ;
les cycles aromatiques des chromophores anthraquinoniques des formules la et Ib peuvent être éventuellement substitués ;
l'électroneutralité du colorant étant assurée par un ou plusieurs anions An-, identiques ou non, cosmétiquement acceptables.

6. Colorant selon la revendication 5, **caractérisé en ce que** les groupements R représentent un atome d'hydrogène, un radical alkyle en C₁-C₂.

7. Colorant selon la revendication 5 ou 6, **caractérisé en ce que** R' représente :
• un atome d'hydrogène,
• un radical alkyle en C₁-C₄, éventuellement substitué,
• une chaîne hydrocarbonée en C₁-C₆ linéaire ou ramifiée, éventuellement substituée, éventuellement interrompue ou terminée par un ou plusieurs groupements: amino, mono ou di-alkylamino éventuellement substitué, ou bien
• le radical COL.

8. Colorant selon l'une quelconque des revendications 5 à 7, **caractérisé en ce que** R₁, indépendamment les uns des autres représentent une chaîne hydrocarbonée en C₁-C₁₀, linéaire ou ramifiée, saturée ou insaturée, pouvant former un ou plusieurs cycles carbonés comportant de 5 ou 6 chaînons, éventuellement condensés avec le cycle aromatique, éventuellement substituée.

9. Colorant selon l'une quelconque des revendications 5 à 8,
**caractérisé en ce que** R₂, indépendamment les uns des autres, représentent:
• une chaîne hydrocarbonée en C₁-C₁₀, linéaire ou ramifiée, saturée ou insaturée, pouvant former un ou plusieurs cycles carbonés comportant de 5 ou 6 chaînons, éventuellement substitué par un ou plusieurs groupements identiques ou différents de préférence choisis parmi hydroxyle, alcoxy en C₁-C₂;
• un groupement hydroxyle,
• un groupement alkyl-oxy en C₁-C₄,
• un groupement alcoxycarbonyle R₁₁O-CO-, dans lequel R₁₁ représente un radical alkyle en C₁-C₂ , un radical alkylcarbonyloxy R₁₂CO-O-, dans lequel R₁₂ représente un radical alkyle en C₁-C₂ ;
• un groupement amino ; un groupement amino substitué par un ou deux radicaux alkyles en C₁-C₄, identiques ou différents, éventuellement porteurs d'au moins un groupement hydroxyle, les deux radicaux alkyles formant éventuellement avec l'atome d'azote auquel ils sont rattachés, un hétérocycle à 5 ou 6 chaînons éventuellement porteur d'un autre hétéroatome identique ou différent de l'azote, par exemple l'oxygène, le soufre
• un groupement alkylcarbonylamino R₁₃CO-NR₁₄-, dans lequel les radicaux R₁₃ et R₁₄ indépendamment l'un de l'autre, représentent un radical alkyle en C₁-C₂ ; un groupement carbamoyle (R₁₅)₂N-CO, dans lequel les radicaux R₁₅ indépendamment l'un de l'autre, identiques ou non, représentent un atome d'hydrogène, un radical alkyle en C₁-C₂ ; un groupement uréido (R₁₆)₂N-CO-NR₁₇-, dans lequel les radicaux R₁₆ et R₁₇, indépendamment l'un de l'autre, représentent un atome d'hydrogène, un radical alkyle en C₁-C₄ ;
• un groupement sulfonamide (R₁₈)₂N-SO₂-, dans lequel les radicaux R₁₈, indépendamment l'un de l'autre, représentent un atome d'hydrogène, un radical alkyle en C₁-C₄;
• un groupement guanidinium (R₂₁)₂N-C(=NH₂⁺)-NR₂₂-, dans lequel les radicaux R₂₁ et R₂₂, indépendamment les uns des autres, représentent un atome d'hydrogène, un radical alkyle en C₁-C₄;
• un groupement alkylsulfonyle R₂₃-SO₂-, dans lequel R₂₃ représente un radical alkyle en C₁-C₄ ;
• un groupement cyano ; un atome d'halogène, de préférence le chlore, le fluor
• un groupement phényle éventuellement substitué.

10. Colorant selon la revendication 9, **caractérisé en ce que** les radicaux R₂ identiques ou non, indépendamment les uns des autres représentent un radical méthyle, éthyle, 2-hydroxyéthyle, 2-méthoxyéthyle, méthylsulfonyle (CH₃SO₂-), méthylcarbonylamino (CH₃CONH-), hydroxyle, amino, méthylamino, diméthylamino, 2-hydroxyéthylamino, bis(2-hydroxyéthyl)amino, méthoxy, éthoxy, phényle.

11. Colorant selon la revendication 9, **caractérisé en ce que** lorsque les radicaux de formules (IIIa) et (IIIb) portent deux radicaux R₂, alors ces radicaux R₂ forment avec les atomes de carbone auxquels ils sont rattachés, un cycle secondaire, aromatique à 6 chaînons, éventuellement substitué par un ou plusieurs groupements identiques ou différents choisis parmi hydroxyle, alkyle en C₁-C₄, alcoxy en C₁-C₄, amino, amino substitué par un ou deux radicaux alkyle en C₁-C₄ identiques ou différents et éventuellement porteurs d'au moins un groupement hydroxyle ou méthylcarbonylamino.

12. Colorant selon la revendication 11, **caractérisé en ce que** les deux radicaux R₂ forment avec les atomes de carbone auxquels ils sont rattachés, un cycle secondaire, aromatique à 6 chaînons, éventuellement substitué par un ou plusieurs hydroxyle, méthoxy, éthoxy, amino, 2-hydroxyéthylamino, diméthylamino, bis-(hydroxyéthyl)amino.

13. Colorant selon l'une quelconque des revendications 5 à 12, **caractérisé en ce que** m représente un entier compris entre 0 et 2.

14. Colorant selon l'une quelconque des revendications 5 à 13, **caractérisé en ce que** e est compris entre 0 et 2.

15. Colorant selon la revendication 14, **caractérisé en ce que** e est égal à 0.

16. Colorant selon l'une quelconque des revendications 5 à 15, **caractérisé en ce que** les radicaux R₃ identiques ou non, représentent:
• un radical alkyle en C₁-C₁₆, éventuellement substitué
• un groupement hydroxyle,
• un groupement alcoxy en C₁-C₂ ; un groupement (poly)hydroxyalcoxy en C₂-C₄ ;
• un groupement alcoxycarbonyle R₃₁O-CO-, dans lequel R₃₁ représente un radical alkyle en C₁-C₄ ; un radical alkylcarbonyloxy R₃₂CO-O-, dans lequel R₃₂ représente un radical alkyle en C₁-C₄ ;
• un radical alkylcarbonyle R₃₃-CO-, dans lequel R₃₃ représente un radical alkyle en C₁-C₄ ;
• un groupement amino ; un groupement amino substitué par un ou deux radicaux alkyle en C₁-C₄, identiques ou différents, éventuellement porteurs d'au moins un groupement hydroxyle ; les deux radicaux alkyle formant éventuellement avec l'atome d'azote auquel ils sont rattachés, un hétérocycle renfermant 1 à 3 hétéroatomes, de préférence 1 à 2 hétéroatomes, choisis parmi N, O, S, de préférence N, comprenant de 5 à 7 chaînons, saturé ou insaturé, aromatique ou non, éventuellement substitué ;
• un groupement alkylcarbonylamino R₃₄CO-NR₃₅-, dans lequel le radical R₃₄ représente un radical alkyle en C₁-C₄ et le radical R₃₅ représente un atome d'hydrogène ou un radical alkyle en C₁-C₄ ;
• un groupement aminocarbonyle (R₃₆)₂N-CO-, dans lequel les radicaux R₃₆ indépendamment l'un de l'autre, représentent un atome d'hydrogène, un radical alkyle en C₁-C₄ ;
• un groupement uréido N(R₃₇)₂-CO-NR₃₈-, dans lequel les radicaux R₃₇ et R₃₈, indépendamment l'un de l'autre, représentent un atome d'hydrogène, un radical alkyle en C₁-C₄ ;
• un groupement aminosulfonyle (R₃₉)₂N-SO₂-, dans lequel les radicaux R₃₉ indépendamment l'un de l'autre, représentent un atome d'hydrogène, un radical alkyle en C₁-C₄ ;
• un groupement alkylsulfonylamino R₄₀SO₂-NR₄₁-, dans lequel les radicaux R₄₀ et R₄₁, indépendamment l'un de l'autre, représentent un atome d'hydrogène, un radical alkyle en C₁-C₄ ;
• un groupement thiol HS- ;
• un groupement alkylthio R₄₂S-, dans lequel le radical R₄₂ représente un radical alkyle en C₁-C₄ ;
• un groupement alkylsulfonyle R₄₄-SO₂-, dans lequel R₄₄ représente un radical alkyle en C₁-C₄ ;
• un groupement cyano ;
• un atome d'halogène tel que le chlore, le fluor.

17. Colorant selon la revendication 16, **caractérisé en ce que** les radicaux R₃, identiques ou différents, indépendamment les uns des autres, représentent :
• un radical alkyle en C₁-C₄ éventuellement substitué par un ou plusieurs radicaux identiques ou différents choisis parmi les radicaux hydroxyle, alkylcarbonylamino en C₁-C₂, amino substitué par deux radicaux alkyles en C₁-C₂ identiques ou différents éventuellement porteurs d'un ou plusieurs groupements identiques ou différents choisis parmi hydroxyle
ou alcoxy en C₁-C₂ ; ces deux radicaux alkyle peuvent éventuellement former avec l'atome d'azote auquel ils sont rattachés, un hétérocycle à 5
ou 6 chaînons, saturé ou non, éventuellement aromatique, choisi de préférence parmi la pyrrolidine, la pipérazine, l'homopipérazine, le pyrrole, l'imidazole, le pyrazole ;
• un radical hydroxyalcoxy en C₂-C₄ ;
• un halogène choisi parmi le chlore ou le fluor ;
• un radical amino ; un radical amino substitué par un ou deux radicaux alkyles en C₁-C₂ , identiques ou différents éventuellement porteurs d'au moins un groupement hydroxyle;
• un radical méthylcarbonylamino ;
• un radical méthylsulfonylamino ;
• un radical hydroxyle ;
• un radical alcoxy en C₁-C₂ ;
• un radical méthylsulfonyle.

18. Colorant selon la revendication 17, **caractérisé en ce que** les radicaux R₃, identiques ou différents, indépendamment les uns des autres, représentent:
• un radical méthyle, éthyle, propyle, 2-hydroxyéthyle,
• un radical méthoxy, éthoxy,
• un radical 2-hydroxyéthyloxy, 3-hydroxypropyloxy,
• un radical 2-méthoxyéthyle ;
• un radical méthylsulfonylamino ;
• un radical amino, méthylamino, diméthylamino, 2-hydroxyéthylamino.
• un radical méthylcarbonylamino ;
• un radical hydroxyle ;
• un atome de chlore ;
• un radical méthylsulfonyle.

19. Colorant selon l'une quelconque des revendications 5 à 18, **caractérisé en ce que** lorsque le coefficient n est supérieur ou égal à 2, alors deux radicaux R₃ adjacents forment avec les atomes de carbone auxquels ils sont rattachés, un cycle secondaire aromatique à 6 chaînons, éventuellement substitué par un ou plusieurs groupements identiques ou différents choisis parmi les groupements hydroxyle, alkyle en C₁-C₄ , alcoxy en C₁-C₄ , (poly)hydroxyalcoxy en C₂-C₄, alkylcarbonylamino en C₁-C₄ , amino, amino substitué par un ou deux radicaux identiques ou différents, alkyle en C₁-C₄ éventuellement porteurs d'au moins un groupement hydroxyle,

20. Colorant selon la revendication 19, **caractérisé en ce que** deux radicaux R₃ adjacents forment avec les atomes de carbone auxquels ils sont rattachés, un cycle secondaire aromatique à 6 chaînons, éventuellement substitué par un ou plusieurs groupements choisis parmi les groupements hydroxyle, méthoxy, éthoxy, 2-hydroxyethyloxy, amino, methylcarbonylamino, (di)2-hydroxyethylamino, -NH-Ph, -NH-Ph-NH₂, -NH-Ph-NHCOCH₃, -NH-Ph-OH, -NH-Ph-OCH₃.

21. Colorant selon l'une quelconque des revendications 5 à 20, **caractérisé en ce que** n représente un entier compris entre 0 et 2.

22. Colorant selon l'une quelconque des revendications 5 à 21, **caractérisé en ce que** R₄, et R₇ représentent :
• un atome d'hydrogène ;
• un radical alkyle en C₁-C₆ éventuellement substitué par un ou plusieurs groupements identiques ou différents de préférence choisis parmi hydroxyle, alcoxy en C₁-C₂;
• un radical aryle, ou arylalkyle, tel que phényle, benzyle, la partie aryle étant éventuellement substituée par un ou plusieurs groupements identiques ou différents de préférence choisis parmi par un atome de chlore, un groupement hydroxyle, un groupement alcoxy en C₁-C₂, un groupement amino, un groupement amino mono ou disubstitué par deux radicaux alkyle en C₁-C₄ identiques ou différents éventuellement porteurs d'au moins un groupement hydroxyle.

23. Colorant selon la revendication 22, **caractérisé en ce que** les radicaux R₄ et R₇, représentent :
• un atome d'hydrogène ;
• un radical alkyle en C₁-C₃ éventuellement substitué, tel que méthyle, éthyle, 2-hydroxyéthyle, 2-méthoxyéthyle ;
• un radical phényle, éventuellement substitué par un ou plusieurs radicaux identiques ou différents choisis parmi les radicaux hydroxyle, alcoxy en C₁-C₂, amino, amino substitués par un ou plusieurs groupements alkyle en C₁-C₄ éventuellement porteurs d'au moins un groupement hydroxyle.

24. Colorant selon la revendication 22, **caractérisé en ce que** les radicaux R₄ et R₇ représentent :
• un atome d'hydrogène ;
• un radical méthyle, éthyle, 2-hydroxyéthyle ;
• un radical phényle, éventuellement substitué par un ou plusieurs radicaux identiques ou différents choisi parmi hydroxyle, méthoxy, amino, (di)méthylamino , (di)(2-hydroxyéthyl)amino.

25. Colorant selon l'une quelconque des revendications 5 à 24, **caractérisé en ce que** R₅ et R₆, indépendamment l'un de l'autre, identiques ou non, représentent:
• un atome d'hydrogène ;
• un radical alkylcarbonyle R₄₅-CO-, dans lequel R₄₅ représente un radical alkyle en C₁-C₄ éventuellement substitué,
• un radical alkyle en C₁-C₆ éventuellement substitué par un ou plusieurs groupements identiques ou non, choisi parmi hydroxyle, alcoxy en C₁-C₂, amino, (di-)alkyle amino en C₁-C₄ ; le radical alkyle peut en outre être substitué par un ou plusieurs groupements identiques ou non, choisi parmi alkylsulfonyle en C₁-C₄ , alkylsulfinyle en C₁-C₄ , alkylcarbonyle en C₁-C₄,
• un radical aryle, ou arylalkyle, tel que phényle, benzyle, la partie aryle étant éventuellement substituée, de préférence par un atome de chlore, un groupement hydroxyle, un groupement alcoxy en C₁-C₄ , un groupement amino, un groupement amino mono- ou di-substitué par deux radicaux identiques ou différents, choisis parmi les radicaux alkyle en C₁-C₄ éventuellement porteurs d'au moins un groupement hydroxyle.

26. Colorant selon la revendication 25, **caractérisé en ce que** les radicaux R₅ et R₆, identiques ou différents, indépendamment l'un de l'autre représentent :
• un atome d'hydrogène ;
• un radical méthylcarbonyle, éthylcarbonyle, propylcarbonyle ;
• un radical alkyle en C₁-C₃ éventuellement substitué, tel que méthyle, éthyle, 2-hydroxyéthyle, 2-méthoxyéthyle ;
• un radical phényle, éventuellement substitué par un ou plusieurs radicaux identiques ou non choisis parmi les radicaux hydroxyle, alcoxy en C₁-C₂, amino, amino substitués par un ou plusieurs groupements alkyle en C₁-C₄ éventuellement porteurs d'au moins un groupement hydroxyle.

27. Colorant selon la revendication 26, **caractérisé en ce que** les radicaux R₅ et R₆, identiques ou différents, indépendamment l'un de l'autre, représentent :
• un atome d'hydrogène ;
• un radical méthyle, éthyle, 2-hydroxyéthyle ;
• un radical méthylcarbonyle, éthylcarbonyle, propylcarbonyle ;
• un radical phényle, éventuellement substitué par un radical hydroxyle, méthoxy, amino, diméthylamino , bis-(2-hydroxyéthyl)amino.

28. Colorant selon l'une quelconque des revendications 5 à 27, **caractérisé en ce que** les radicaux R₅ et R₆ forment ensemble avec l'atome d'azote auquel chacun est rattaché, un hétérocycle renfermant 1 à 2 hétéroatomes, choisis parmi N, O, S, de préférence N, comprenant de 5 à 7 chaînons, saturé ou insaturé, aromatique ou non, éventuellement substitué.

29. Colorant selon la revendication 28, **caractérisé en ce que** l'hétérocycle comprenant de 5 à 7 chaînons représente un hétérocycle de type pipéridine, pipérazine, homopipérazine, pyrrole, imidazole, pyrazole éventuellement substitué par un ou plusieurs radicaux identiques ou non choisis parmi les radicaux hydroxyle, alcoxy en C₁-C₂, amino, amino substitués par un ou plusieurs groupements alkyle en C₁-C₄ éventuellement porteurs d'au moins un groupement hydroxyle.

30. Colorant selon la revendication 29, **caractérisé en ce que** les radicaux R₅ et R₆ forment ensemble avec l'atome d'azote auquel chacun est rattaché, un hétérocycle comprenant de 5 à 7 chaînons choisi parmi les hétérocycles suivants : pipéridine, 2-(2-hydroxy éthylpipéridine), 4-(aminométhyl)pipéridine, 4-(2-hydroxyéthyl) pipéridine, 4-(diméthylamino)pipéridine, pipérazine, 1-méthyl pipérazine, 1-(2-hydroxyéthyl)pipérazine, 1-(2-aminoéthyl)pipérazine, 1-hydroxyéthyléthoxy pipérazine, homopipérazine, 1-méthyl-1,4-perhydrodiazépine, pyrrole, 1,4-diméthylpyrrole, 1-méthyl-4-éthylpyrrole, 1-méthyl-4-propylpyrrole.

31. Colorant selon l'une quelconque des revendications 5 à 30, **caractérisé en ce que** les radicaux R₅ et R₆ peuvent former, avec l'atome de carbone du cycle aromatique éventuellement substitué par un hydroxyle et adjacent à celui auquel -NR₅R₆, est rattaché, un hétérocycle saturé à 5 ou 6 chaînons ou des hétérocycles condensés saturés à 5 ou 6 chaînons.

32. Colorant selon la revendication 31, **caractérisé en ce que** lorsque W représente le groupement -NR₅R₆, et que la liaison b*se trouve en para de -NR₅R₆, alors avec le noyau aromatique éventuellement substitué par un hydroxyle a la structure suivante : 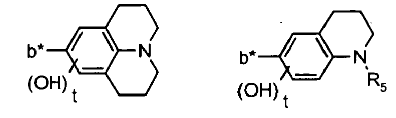 dans lesquelles t a la valeur de 0 ou 1
R₅ est tel que défini dans les revendications 25 à 31,
b* est tel que défini dans la revendication 5.

33. Colorant selon l'une quelconque des revendications 5 à 32, **caractérisé en ce que** R" représente un atome d'hydrogène ou un radical alkyle en C₁-C₁₀, éventuellement substitué.

34. Colorant selon l'une quelconque des revendications 5 à 33, **caractérisé en ce que** R"' représente un atome d'hydrogène ou un radical alkyle en C₁-C₁₀, éventuellement substitué.

35. Colorant selon l'une quelconque des revendications 5 à 34, **caractérisé en ce que** la liaison a* se situant :
• soit sur l'un des atomes d'azote quaternisé ou non des formules (IIIa)
ou (IIIb),
• soit sur l'atome d'azote portant les radicaux R₅ et R₆
• soit sur l'un des atomes du groupement R₅, R₆ ou R₇ .

36. Colorant selon l'une quelconque des revendications 5 à 35 , **caractérisé en ce que** L₁ et/ou L₂ est/sont un bras de liaison non cationique.

37. Colorant selon la revendication 36, **caractérisé en ce que** L₁ et/ou L₂ est choisi parmi:
• un radical alkylène en C₁-C₂₀, éventuellement substitué, éventuellement interrompu par un (hétéro)cycle saturé ou insaturé, aromatique ou non, comprenant de 3 à 7 chaînons, éventuellement substitué, éventuellement condensé ; le dit radical alkylène étant éventuellement interrompu par un ou plusieurs hétéroatomes ou groupements comprenant au moins un hétéroatome de préférence l'oxygène, l'azote, le soufre, tels que -CO-, - SO₂- ou leurs combinaisons ; le bras de liaison L₁ et/ou L₂ ne comprenant pas de fonction azo, nitro, nitroso, peroxo.
L₂ peut également représenter une liaison covalente, lorsque R' est différent de COL.

38. Colorant selon la revendication 37, **caractérisé en ce que** L₁ et L₂ indépendemment l'un de l'autre représentent un radical alkylène choisi parmi méthylène, éthylène, propylène linéaire ou ramifié, butylène linéaire ou ramifié, pentylène linéaire ou ramifié, hexylène linéaire ou ramifié, éventuellement substitués et/ou interrompus par un
ou plusieurs hétéroatomes ou groupements comprenant au moins un hétéroatome de préférence l'oxygène, l'azote, le soufre, tels que -CO-, - SO₂- ou leurs combinaisons ; le bras de liaison L₁ et/ou L₂ ne comprenant pas de fonction azo, nitro, nitroso, et peroxo.

39. Colorant selon la revendication 38, **caractérisé en ce que** L₁ et L₂ indépendemment l'un de l'autre représentent un radical alkyléne substituée par des substituants, identiques ou différents choisis parmi l'hydroxyle, l'alcoxy en C₁-C₂, le dialkylamino en C₁-C₂, l'alkyl(C₁-C₄)carbonyle, l'alkyl(C₁-C₄)sulfonyle.

40. Colorant selon la revendication 37, **caractérisé en ce que** le cycle ou l'hétérocycle, saturé ou insaturé, aromatique ou non, pouvant interrompre le radical alkylène du bras de liaison L₁ et/ou L₂ est choisi parmi le phénylène, naphtylène, phénanthrylène, triazinyle, pyrimidinyle, pyridinyle, pyridazinyle, quinoxalinyle, cyclohexyle.

41. Colorant selon l'une quelconque des revendications 36 à 40, **caractérisé en ce que** L₁ et/ou L₂ indépendamment l'un de l'autre sont choisis parmi les radicaux suivants : 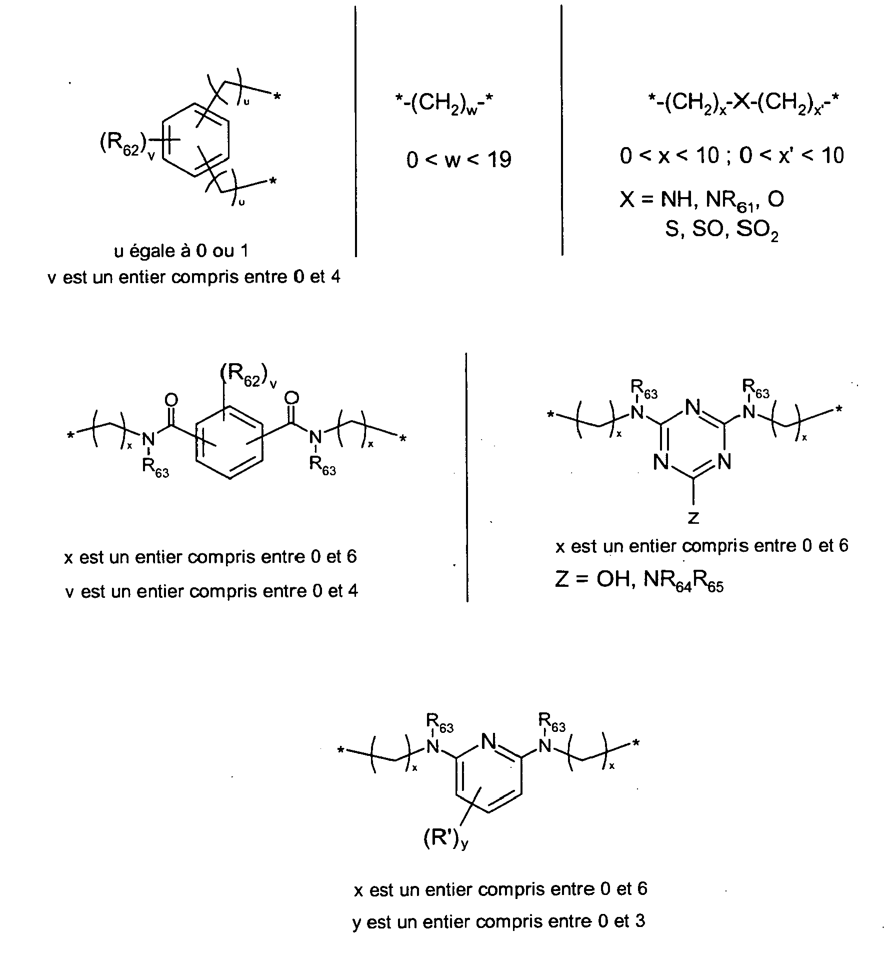 dans lesquelles :
• R₆₂ et R' a la même définition que R₃ ;
• R₆₃ représente un hydrogène, un radical alkyle en C₁-C₄ ;
• R₆₄ et R₆₅ représentent, indépendamment l'un de l'autre, un atome d'hydrogène, un radical alkyle en C₁-C₈ éventuellement substitué par un
ou plusieurs radicaux identiques ou différents choisis parmi hydroxyle, alcoxy en C₁-C₂, (poly)hydroxyalcoxy en C₂-C₄, amino, (di-) alkylamino en C₁-C₂ ou aryle éventuellement substitué,
• * représentent les extrémités des bras de liaison L₁ et/ou L₂,
• R₆₁ représente un radical alkyle en C₁-C₄ éventuellement substitué par un ou plusieurs radicaux identiques ou différents choisis parmi hydroxyle, alcoxy en C₁-C₂, (poly)hydroxyalcoxy en C₂-C₄, amino, (di-) alkylamino en C₁-C₂ ou aryle éventuellement substitué.

42. Colorant selon l'une quelconque des revendications à 35, **caractérisé en ce que** l'un au moins des bras de liaison L₁ et/ou L₂ représente un bras de liaison portant au moins une charge cationique.

43. Colorant selon la revendication 42, **caractérisé en ce que** L1 et/ou L2 indépendamment l'un de l'autre représente un radical alkylène en C₂-C₄₀, portant au moins une charge cationique, éventuellement substitué et/ou éventuellement interrompu par un ou plusieurs (hétéro)cycle saturé ou non, aromatique ou non, identique ou différent, comprenant de 5 à 7 chaînons et/ou éventuellement interrompu par un ou plusieurs hétéroatomes ou groupements comprenant au moins un hétéroatome ou leurs combinaisons, comme par exemple l'oxygène, l'azote, le soufre, un groupement -CO-, -SO₂- ou leurs combinaisons ; le bras de liaison L₁ et/ou L₂ ne comprenant pas de fonction azo, nitro, nitroso, et peroxo.

44. Colorant selon la revendication 43, **caractérisé en ce que** L₁ et/ou L₂ cationique représente un radical alkylène en C₂-C₂₀ :
• interrompu par au moins un groupement correspondant aux formules suivantes : 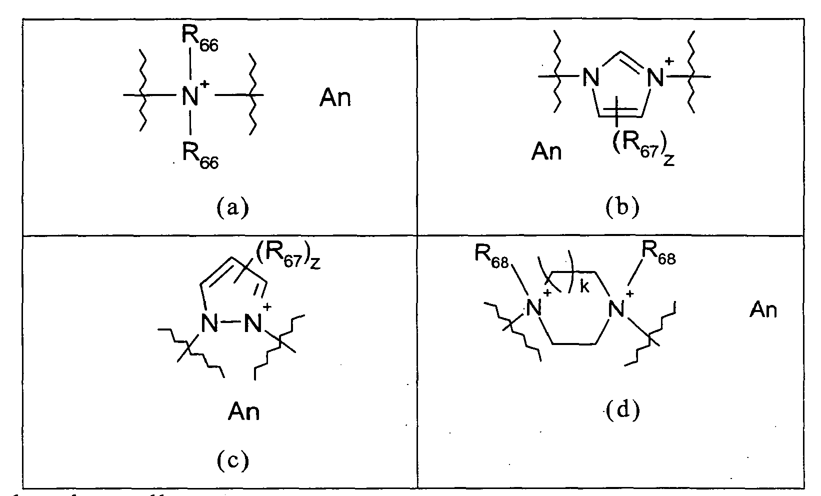
dans lesquelles :
- R₆₆ et R₆₈ indépendamment l'un de l'autre, représentent un radical alkyle en C₁-C₈ ; un radical monohydroxyalkyle en C₁-C₆ ; un radical polyhydroxyalkyle en C₂-C₆ ; un radical alcoxy(C₁-C₆)alkyle en C₁-C₆; un radical aryle tel que phényle éventuellement substitué; un radical arylalkyle tel que benzyle éventuellement substitué; un radical aminoalkyle en C₁-C₆; un radical aminoalkyle en C₁-C₆ dont l'amine est substituée par un ou deux radicaux alkyle C₁-C₄ identiques ou différents, un radical alkyl(C₁-C₆)sulfonyle,
- deux radicaux R₆₆ peuvent former ensemble, avec l'atome d'azote auxquels ils sont rattachés, un cycle saturé à 6 chaînons,
- R₆₇, identiques ou différents, représentent un atome d'halogène ; choisi parmi le brome, le chlore ou le fluor, un radical alkyle en C₁-C₆, un radical monohydroxyalkyle en C₁-C₆, un radical polyhydroxyalkyle en C₂-C₆, un radical alcoxy en C₁-C₆, un radical (di-)alkylamino en C₁-C₄, un radical hydroxycarbonyle, un radical alkylcarbonyle en C₁-C₆, un radical thioalkyle en C₁-C₆, un radical alkyl(C₁-C₆)thio, un radical alkyl(C₁-C₆)sulfonyle, un radical benzyle éventuellement substitué, un radical phényle éventuellement substitué par un ou plusieurs radicaux choisis parmi les radicaux méthyle, hydroxyle, amino, méthoxy,
- An est un anion ou un mélange d'anions, organiques ou minéraux
- z est un nombre entier compris entre 1 et 3 ; si z est inférieur à 3, alors les atomes de carbone non substitués portent un atome d'hydrogène,
- k est un entier égal à 1 ou 2 de préférence égal à 1 ;
• éventuellement interrompu par un ou plusieurs hétéroatomes ou groupements comprenant au moins un hétéroatome ou leurs combinaisons, comme par exemple l'oxygène, l'azote, le soufre, un groupement -CO-, un groupement -SO₂- ; à la condition qu'il n'y ait pas de groupe ou liaison nitro, nitroso, peroxo dans le bras de liaison L₁ et/ou L₂;
• et éventuellement substitué par un ou plusieurs radicaux choisis parmi les radicaux hydroxyle, alcoxy en C₁-C₂, (poly)-hydroxyalcoxy en C₂-C₄, amino substitué par un ou plusieurs groupements alkyle linéaires ou ramifiés en C₁-C₂ éventuellement porteurs d'au moins un groupement hydroxyle.

45. Colorant selon la revendication 44, **caractérisé en ce que** les radicaux R₆₆ et R₆₈ des formules (a) et (d), séparément, sont choisis parmi un radical alkyle en C₁-C₆, un radical monohydroxyalkyle en C₁-C₄, un radical polyhydroxyalkyle en C₂-C₄, un radical alcoxy(C₁-C₆)alkyle en C₂-C₄, un radical diméthylaminoalkyle en C₂-C₆.

46. Colorant selon la revendication 45, **caractérisé en ce que** les radicaux R₆₆ et R₆₈ séparément représentent un radical méthyle, éthyle, 2-hydroxyéthyle.

47. Colorant selon la revendication 44, **caractérisé en ce que** le radical R₆₇ des formules (b) et (c) représente un atome d'halogène choisi parmi le chlore et le fluor, un radical alkyle en C₁-C₆, un radical monohydroxylalkyle en C₁-C₄, un radical alcoxy en C₁-C₄, un radical hydroxycarbonyle, un radical thioalkyle en C₁-C₆, un radical amino disubstitué par un radical alkyle en C₁-C₄.

48. Colorant selon la revendication 47, **caractérisé en ce que** le radical R₆₇ des formules (b) et (c) représente un atome de chlore, un méthyle, un éthyle, un 2-hydroxyéthyle, un methoxy, un hydroxycarbonyle, un dimethylamino.

49. Colorant selon la revendication 44, **caractérisé en ce que** z des formules (b) et (c) est égal à 0.

50. Colorant selon l'une quelconque des revendications précédentes, **caractérisé en ce qu'**il répond aux formules générales suivantes :
- Anthraquinones reliées à deux chromophores azoïques en série azo-imidazolium : 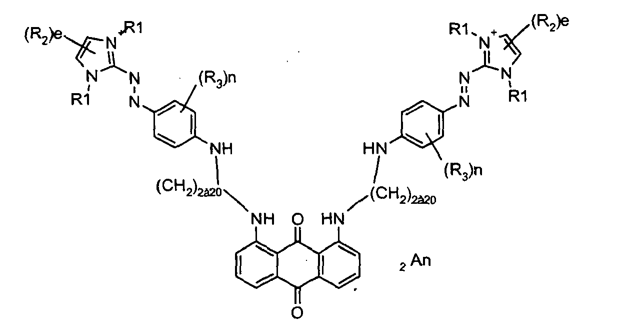
- Anthraquinones reliées à un chromophore azoïque en série azo-imidazolium : 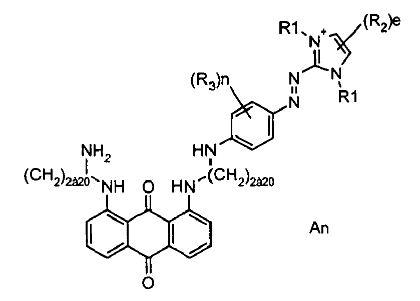
- Anthraquinones reliées à deux chromophores azoïques en série azo-imidazolium : 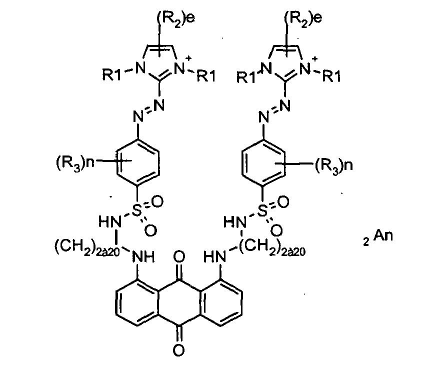
- Anthraquinones reliées à deux chromophores azoïques en série 3-azopyridinium : 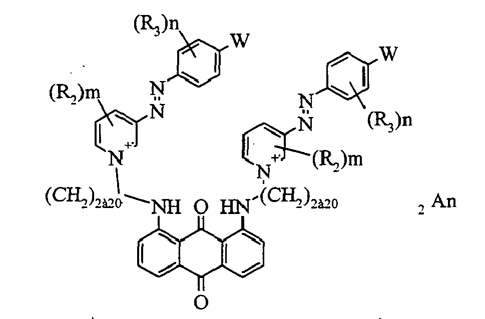
- Anthraquinones reliées à deux chromophores hydrazones en série 4-pyridinium : 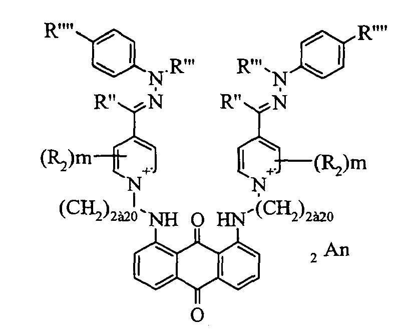
- Anthraquinones reliées à deux chromophores hydrazones en série 4-pyridinium - variante pour laquelle le bras de liaison est cationique 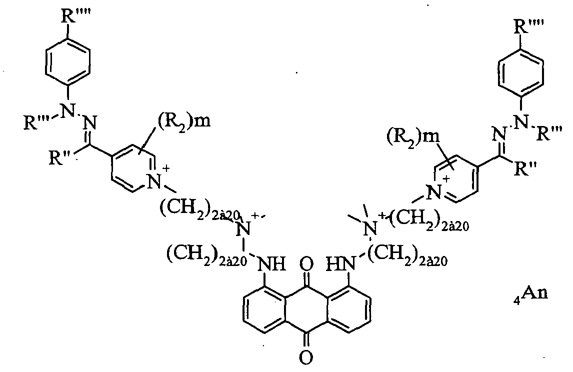
- Anthraquinones reliées à un chromophore hydrazone en série 4-pyridinium - variante pour laquelle le bras de liaison est cationique 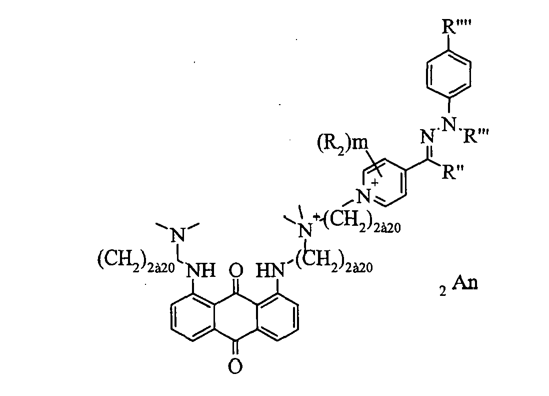
- Anthraquinones reliées à un chromophore hydrazone en série 4-quinolinium - variante pour laquelle le bras de liaison est cationique : 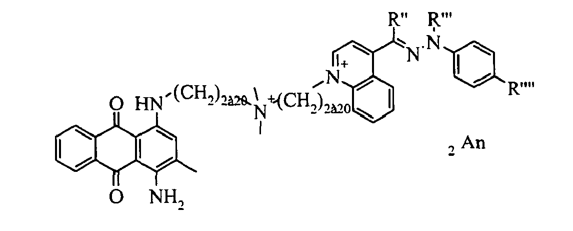
- Anthraquinones reliées à un chromophore hydrazone en série 4-pyridinium - variante pour laquelle le bras de liaison est cationique : 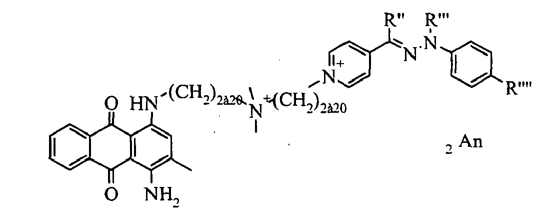
- Anthraquinones reliées à un chromophore azoïque en série azo-imidazolium : 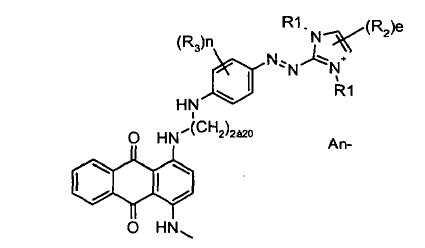
ou l'un de leurs sels ou solvates physiologiquement acceptables.

51. Colorant selon l'une quelconque des revendications précédentes, **caractérisé en ce qu'**il est choisi parmi les composés de formule suivante :
Anthraquinones reliées à deux chromophores azoïques en série azo-imidazolium : 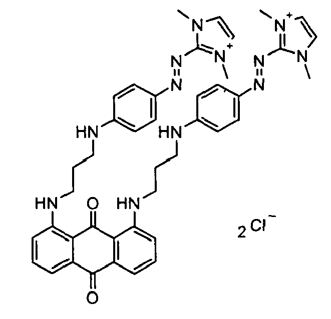
Anthraquinones reliées à un chromophore azoïque en série azo-imidazolium : 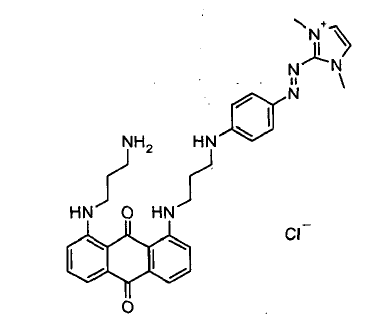
Anthraquinones reliées à deux chromophores azoïques en série azo-imidazolium : 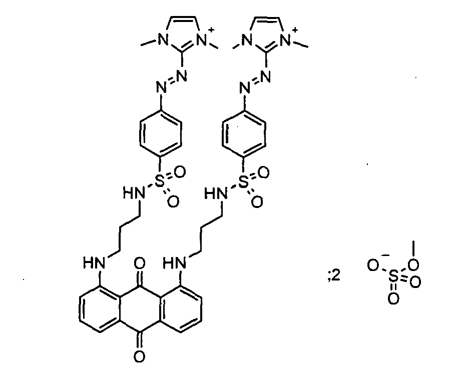
Anthraquinones reliées à deux chromophores azoïques en série 3-azopyridinium : 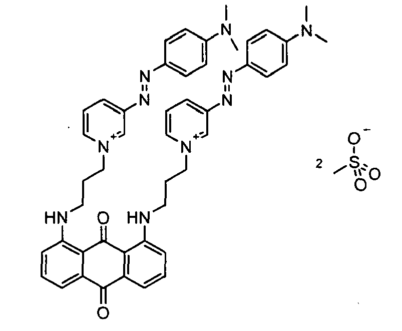
Anthraquinones reliées à deux chromophores hydrazones en série 4-pyridinium : 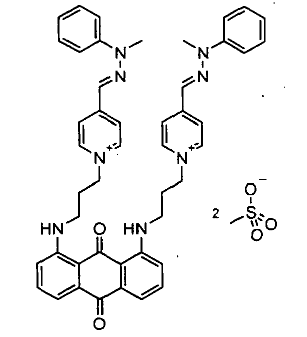
Anthraquinones reliées à deux chromophores hydrazones en série 4-pyridinium - variante pour laquelle le bras de liaison est cationique 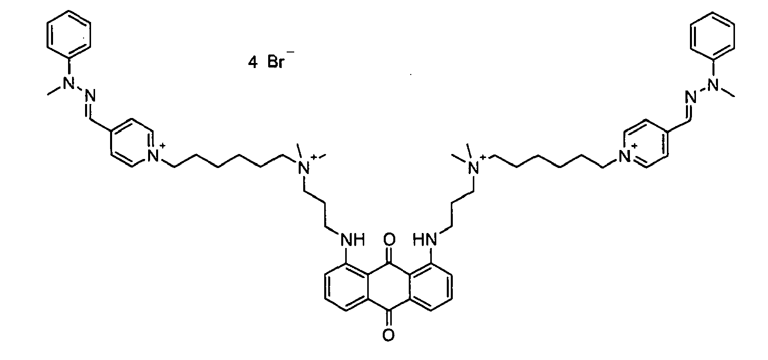
Anthraquinones reliées à un chromophore hydrazone en série 4-pyridinium - variante pour laquelle le bras de liaison est cationique 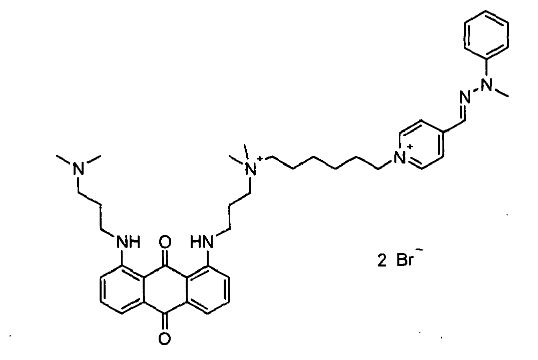
Anthraquinones reliées à un chromophore hydrazone en série 4-quinolinium - variante pour laquelle le bras de liaison est cationique : 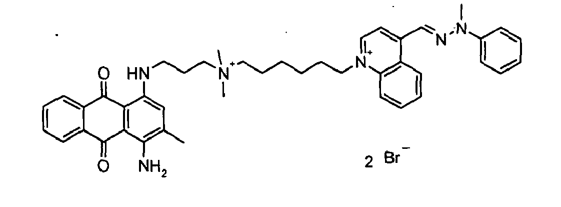
Anthraquinones reliées à un chromophore hydrazone en série 4-pyridinium - variante pour laquelle le bras de liaison est cationique : 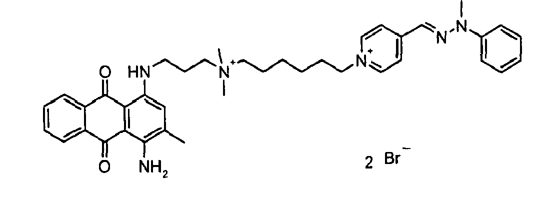
Anthraquinones reliées à un chromophore azoïque en série azo-imidazolium :

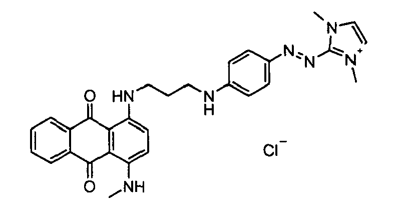

52. Composition tinctoriale comprenant, dans un milieu approprié pour la teinture des fibres kératiniques, notamment humaines, au moins un colorant mixte tel que défini dans l'une quelconque des revendications 1 à 51.

53. Composition selon la revendication 52, **caractérisée en ce que** la teneur en colorant mixte varie de 0,001 à 20% en poids, plus particulièrement, de 0,005 à 10% en poids, et de préférence, de 0,01 et 5% en poids, par rapport au poids total de la composition.

54. Composition selon la revendication 52 ou 53, **caractérisée en ce qu'**elle comprend un ou plusieurs colorants directs additionnels autres que ledit ou lesdits colorants mixtes.

55. Composition selon la revendication précédente, **caractérisée en ce que** la teneur en chacun des colorants directs additionnels est varie de 0,001 à 10 % en poids par rapport au poids total de la composition tinctoriale.

56. Composition selon l'une quelconque des revendications 52 à 55, **caractérisée en ce qu'**elle comprend au moins une base d'oxydation choisie parmi les paraphénylènediamines, les bis-phénylalkylènediamines, les para-aminophénols, les ortho-aminophénols, les bases hétérocycliques et leurs sels d'addition.

57. Composition selon l'une quelconque des revendications 52 à 56, **caractérisée en ce qu'**elle comprend au moins un coupleur choisi parmi les métaphénylènediamines, les méta-aminophénols, les métadiphénols, les coupleurs naphtaléniques, les coupleurs hétérocycliques et leurs sels d'addition.

58. Composition selon l'une quelconque des revendications 56 ou 57, **caractérisée en ce que** la teneur de chacune des bases d'oxydation est varie de 0,001 à 10 % en poids par rapport au poids total de la composition tinctoriale et la teneur de chacun des coupleurs varie de 0,001 à 10 % en poids par rapport au poids total de la composition tinctoriale.

59. Composition selon l'une quelconque des revendications 52 à 58, **caractérisée en ce que** la composition comprend au moins un agent oxydant.

60. Composition selon la revendication précédente, **caractérisée en ce que** l'agent oxydant est choisi parmi le peroxyde d'hydrogène, les peroxydes de métaux alcalins ou alcalino-terreux, le peroxyde d'urée, les bromates ou ferricyanures de métaux alcalins, les persels, les enzymes telles que les peroxydases et les oxydo-réductases à deux ou à quatre électrons, et de préférence le peroxyde d'hydrogène.

61. Composition selon l'une quelconque des revendications 52 à 60, **caractérisée en ce que** le pH est compris entre 8 et 11.

62. Procédé de coloration de fibres kératiniques, notamment humaines, consistant à mettre en oeuvre les étapes suivantes :
- on applique la composition selon l'une quelconque des revendications 52 à 61, sur de fibres sèches ou non, éventuellement en présence d'au moins un agent oxydant,
- on laisse poser pendant une durée suffisante pour obtenir la coloration souhaitée,
- on rince éventuellement les fibres,
- on lave les fibres et on les rince,
- on sèche ou on laisse sécher les fibres.

63. Dispositif à plusieurs compartiments dans lequel au moins un premier compartiment contient une composition tinctoriale comprenant au moins un colorant mixte telle que décrit dans l'une des revendications 52 à 61, et éventuellement au moins un colorant direct additionnel différent du ou des colorants mixtes, éventuellement au moins une base d'oxydation, éventuellement au moins un coupleur, et un autre compartiment contenant un agent oxydant.

64. Utilisation d'au moins colorant mixte tel que défini à l'une quelconque des revendications 1 à 51 pour la coloration des fibres kératiniques, telles que les cheveux.

65. Utilisation de la composition tinctoriale telle que définie à l'une quelconque des revendications 52 à 61 pour la coloration des fibres kératiniques, telles que les cheveux.
